# EUROPEAN PATENT SPECIFICATION

(11) **EP 3 216 482 B1**
(45) Date of publication and mention of the grant of the patent: **20.03.2019**
(21) Application number: 15856264.5
(22) Date of filing: 06.11.2015
(51) Int. Cl.: A61M 37/00, B29C 33/38, B29C 59/02, B29C 33/44, B29C 33/00, B29C 59/04, B29L 31/00

(54) **TRANSDERMAL DELIVERY DEVICE AND METHOD FOR MANUFACTURING TRANSDERMAL DELIVERY DEVICE**
TRANSDERMALE VERABREICHUNGSVORRICHTUNG UND VERFAHREN ZUR HERSTELLUNG EINER TRANSDERMALEN VERABREICHUNGSVORRICHTUNG
DISPOSITIF D'ADMINISTRATION TRANSDERMIQUE ET PROCÉDÉ DE FABRICATION DE DISPOSITIF D'ADMINISTRATION TRANSDERMIQUE

(30) Priority: 06.11.2014 JP 2014226366; 13.01.2015 JP 2015004382
(43) Date of publication of application: 13.09.2017
(73) Proprietor: Toppan Printing Co., Ltd., Tokyo 110-0016 (JP)
(72) Inventor: KATO, Hiroyuki, Tokyo 110-0016 (JP); ASAI, Ryoichi, Tokyo 110-0016 (JP); SUMIDA, Tomoya, Tokyo 110-0016 (JP)
(74) Representative: Cabinet Plasseraud
(86) International application number: PCT/JP2015/081320
(87) International publication number: WO 2016/072493

(56) References cited:
- JP-A- 2006 513 811
- JP-A- 2013 111 104
- JP-A- 2014 113 318
- JP-A- 2014 166 352
- US-A1- 2002 115 957
- US-A1- 2004 087 992

## Description

### [Technical Field]

The present invention relates to transdermal administration devices and methods for producing transdermal administration devices.

### [Background Art]

Intradermal sites which are located inside the skin have a high immune response in the body. Since intradermal drug administration is expected to reduce the drug dosage required to achieve an administration effect compared with hypodermic administration by which a drug is delivered to a site under the skin, technical development for intradermal drug administration has been conducted.

For example, intradermal drug administration includes a Mantoux method by which a drug is delivered into an upper site in the skin by using an injection needle, and iontophoresis by which an ionized drug is intradermally infiltrated by applying a weak electric current to the skin. Other examples include jet injection by which a drug is intradermally delivered by means of hydraulic pressure, and a drug delivery method by which a drug is intradermally delivered via a hole created by a microneedle having a fine needle.

In the Mantoux method, which uses an injection needle for drug administration, many patients feel fear of injection needles in addition to pain caused by piercing using an injection needle. Further, iontophoresis and jet injection, which do not involve pain or fear caused by injection needles, require a large device for drug administration.

On the other hand, a needle-shaped projection of a microneedle is so small that patients would not feel pain or fear, and a method using a microneedle can perform drug administration without using a large device (for example, see PTLs 1 to 3).

### [Citation List]

### [Patent Literature]

[PTL 1] WO2008/013282
[PTL 2] WO2008/004597
[PTL 3] WO2008/020632

Further, US2004/087992 A1 discloses a closest prior art document with regard to the subject-matter of the independent claims. US202/115957 A1 shows another example of a prior art device and JP 2014113318 A describes a known method of producing such a device.

### [Summary of the Invention]

### [Technical Problem]

However, since a needle-shaped projection of a microneedle has an elongated shape extending from a surface of a plate-shaped substrate, it does not have a sufficient strength against a force in the lateral direction which is parallel to the surface of the substrate. When the projection pierces the skin, force in the lateral direction is inevitably applied to the projection since the surface of the skin is not flat. Accordingly, if lateral force is excessively applied to the projection, the projection is bent or collapsed, leading to decrease in piercing properties of the projection.

Therefore, in transdermal administration devices such as microneedles having a fine projection to create a passage for intradermal drug administration, there is a need for a device having a projection which is not easily deformed compared with that of the microneedle.

The present invention has an object of providing a transdermal administration device that reduces deformation of a projection and a method for producing the transdermal administration device.

### [Solution to Problem]

A transdermal administration device that solves the above problem includes an administration section including a substrate having a first surface and a second surface which is a surface opposite from the first surface, and a projection which protrudes from the first surface, wherein the projection has a shape extending along the first surface, and includes: one linear top edge which is located away from the first surface, the top edge having a first end and a second end; two main lateral faces which have the top edge in common with each other, the two main lateral faces having lateral edges, each lateral edge individually connecting the first end of the top edge and the first surface; and an auxiliary lateral face which has the lateral edges in common with the respective main lateral faces and forms one corner together with the two main lateral faces, an angle made between the lateral edge and the top edge on the main lateral face is an obtuse angle, and an angle made between the two lateral edges on the auxiliary lateral face is an acute angle.

According to the above configuration, in piercing of the projection into the target, a corner of the projection formed by the two main lateral faces and the auxiliary lateral face is first pierced into the target. At this time, the corner is subject to an external force having a large component directed in the first direction, which is an extending direction of the projection. Since an angle formed between the top edge and the lateral edge on the main lateral face is an obtuse angle, the corner has high strength against the external force acting on the corner, compared with a case where the angle is a right angle or an acute angle. On the other hand, since the angle made between the two lateral edges on the auxiliary lateral face is an acute angle, the corner has a sharp shape when viewed in the first direction compared with a case where the angle is a right angle or an acute angle. As a result, since the sharpness of the corner viewed in the first direction is prevented from being excessively reduced and the strength of the corner against the external force in the first direction can be enhanced, deformation of the projection can be reduced.

In the above transdermal administration device, the auxiliary lateral face may include a base side located within the first surface, and an aspect ratio which is a ratio of a height of the auxiliary lateral face to a length between both ends of the base side may be larger than 1.

According to the above configuration, the aspect ratio of the auxiliary lateral face is larger than 1. Accordingly, the auxiliary lateral face has a sharp shape compared with a case where the aspect ratio of the auxiliary lateral face is not more than 1. As a result, the corner has higher sharpness, which facilitates piercing of the projection.

In the above transdermal administration device, the auxiliary lateral face may be a triangular flat surface having an apex made by the first end of the top edge.

According to the above configuration, the auxiliary lateral face is a triangular flat surface, and accordingly, designing of an angle made between the top edge and the lateral edge and designing of an angle of the corner viewed in the first direction are facilitated.

In the above transdermal administration device, the auxiliary lateral face may be a curved surface which curves inward to the projection.

According to the above configuration, the auxiliary lateral face which constitutes the corner is pierced into the target by digging into the target. Accordingly, the projection can be easily pierced into the skin compared with a case where the auxiliary lateral face is a flat surface.

In the above transdermal administration device, the auxiliary lateral face may be a first auxiliary lateral face, the lateral edge may be a first lateral edge, the two main lateral faces may have lateral edges, each lateral edge individually connecting the second end of the top edge and the first surface, and the transdermal administration device may further includes a second auxiliary lateral face which has the second lateral edges in common with the respective main lateral faces and forms one corner together with the two main lateral faces.

In the above transdermal administration device, a direction along which the projection extends may be a first direction, the top edge may extend along the first direction, the substrate may have a shape extending along the first direction when viewed in a direction perpendicular to the first surface, the transdermal administration device may include a plurality of the projections, and the plurality of projections may include the plurality of the projections disposed at different positions in the first direction on the first surface.

According to the above configuration, the direction in which a user of the transdermal administration device can easily press the substrate against the target matches the direction in which the projection should be pressed against the target. Therefore, the projection can be easily pierced into the target.

In the above transdermal administration device, a direction along which the projection extends may be the first direction, the top edge may extend along the first direction, the transdermal administration device may include the plurality of the projections, the plurality of projections may include the plurality of the projections disposed at different positions in the first direction on the first surface, the transdermal administration device may further include an adhesive sheet having an adhesive surface, the adhesive surface may be bonded to the second surface, and the adhesive surface may have a shape extending along the first direction when viewed in the direction perpendicular to the first surface and protrude outward from the substrate.

According to the above configuration, the direction in which a user of the transdermal administration device can easily press the adhesive surface against the target matches the direction in which the projection of the administration section bonded to the adhesive surface should be pressed against the skin. Therefore, the projection can be easily pierced into the target.

In the above transdermal administration device, the substrate may have a shape extending along the first direction when viewed in the direction perpendicular to the first surface.

According to the above configuration, both the direction in which a user of the transdermal administration device can easily press the substrate against the target and the direction in which a user can easily press the adhesive surface against the target match the direction in which the projection should be pressed against the target. Therefore, the projection can be easily pierced into the target.

A method of producing a transdermal administration device that solves the above problem includes the steps of: forming a molded product by filling a recess of an intaglio plate with a forming material of the administration section, the recess being formed to conform with a shape of the projection; and removing the molded product from the intaglio plate so that removal is carried out in an extending direction of the recess when viewed in a direction perpendicular to a surface of the intaglio plate.

According to the above method, the above transdermal administration device can be produced. This transdermal administration device can reduce deformation of the projection as described above.

Further, methods for producing a microneedle have been proposed in which an administration section is produced by transfer molding using an intaglio plate. For example, the intaglio plate is filled with thermoplastic resin to produce a molded product, and the molded product is removed from the intaglio plate to form an administration section. However, these production methods may have a phenomenon that the resin is adhered to the intaglio plate during removal of the molded product from the intaglio plate. If this phenomenon occurs, the precision of shape-transfer from the intaglio plate to the removed molded product is reduced. Moreover, even if a material other than a thermoplastic resin is used as a forming material of the administration section, the forming material may be partially adhered to the intaglio plate during removal of the molded product from the intaglio plate, which may decrease the precision of shape-transfer from the intaglio plate to the removed molded product.

On the other hand, according to the above method for producing a transdermal administration device, the molded product is easily removed from the intaglio plate since the molded product is removed from the intaglio plate in the extending direction of the recess when viewed in the direction perpendicular to the surface of the intaglio plate. As a result, the forming material is prevented from being partially adhered to the intaglio plate. Accordingly, the precision of shape-transfer from the intaglio plate to the removed molded product is improved.

### [Advantageous Effects of Invention]

According to the present invention, deformation of the projection can be reduced.

### [Brief Description of the Drawings]

Fig. 1 is a perspective view which shows a perspective structure of a transdermal administration device of a first embodiment.
Fig. 2 is a perspective view which shows a perspective structure of a projection of the first embodiment.
Fig. 3 is a view which shows each structure of a main lateral face and an auxiliary lateral face of a projection of the first embodiment when viewed in a direction perpendicular to each surface.
Fig. 4 is a plan view which shows a plan structure of a transdermal administration device of the first embodiment.
Fig. 5 is a view which schematically shows a position of the projection along with a skin surface immediately before the transdermal administration device of the first embodiment is pierced into the skin of an administration target.
Fig. 6 is a view which schematically shows a position of the projection along with a skin surface when the transdermal administration device of the first embodiment is being pierced into the skin of an administration target.
Fig. 7 is a view which schematically shows a relationship between an array of a plurality of projections and change in position where a pressing force is applied while the transdermal administration device of the first embodiment is pressed against the skin.
Fig. 8 is a perspective view which shows a perspective structure of a projection of a second embodiment.
Fig. 9 is a view which shows each structure of a main lateral face and an auxiliary lateral face of a projection of the second embodiment when viewed in a direction perpendicular to each surface.
Fig. 10 is a perspective view which shows a perspective structure of a projection of a modified example.
Fig. 11 is a view which shows each structure of a main lateral face and an auxiliary lateral face of a projection of the modified example when viewed in a direction perpendicular to each surface.
Fig. 12 is a perspective view which shows a perspective structure of a projection of a modified example.
Fig. 13 is a cross sectional view which shows a cross sectional structure of a transdermal administration device package of a third embodiment, and includes (A) which shows a cross sectional structure of a transdermal administration device package which includes a transdermal administration device having one projection, and (B) which shows a cross sectional structure of a transdermal administration device package which includes a transdermal administration device having a plurality of projections.
Fig. 14 includes (A) which is a perspective view that shows a perspective structure of the transdermal administration device package of the third embodiment, and (B) which is a plan view that shows a plan structure of the transdermal administration device package of the third embodiment.
Fig. 15 is a schematic view which shows an intaglio plate used in a method for producing a transdermal administration device of a fourth embodiment and a molded product removed from the intaglio plate.
Fig. 16 is a view which shows part of a production process of the transdermal administration device of the fourth embodiment, and shows the intaglio plate.
Fig. 17 is a view which shows part of the production process of the transdermal administration device of the fourth embodiment, and shows that the forming material of the administration section is provided into the intaglio plate.
Fig. 18 is a view which shows part of the production process of the transdermal administration device of the fourth embodiment, and shows that the forming material of the administration section is filled into the intaglio plate to produce the molded product.
Fig. 19 is a view which shows part of the production process of the transdermal administration device of the fourth embodiment, and shows that the molded product is removed from the intaglio plate.
Fig. 20 is a view which shows part of the production process of the transdermal administration device of the fourth embodiment, and shows the administration section which is the removed molded product.
Fig. 21 is a view which shows part of the production process of the transdermal administration device of the fourth embodiment, and shows the intaglio plate.
Fig. 22 is a view which shows part of the production process of the transdermal administration device of the fourth embodiment, and shows that the forming material of the administration section is supplied into the intaglio plate.
Fig. 23 is a view which shows part of the production process of the transdermal administration device of the fourth embodiment, and shows that the forming material of the administration section is filled into the intaglio plate to produce the molded product.
Fig. 24 is a view which shows part of the production process of the transdermal administration device of the fourth embodiment, and shows that the molded product is removed from the intaglio plate.
Fig. 25 is a view which shows part of the production process of the transdermal administration device of the fourth embodiment, and shows the administration section which is the removed molded product.
Fig. 26 is a view which shows a flow of a production process of a transdermal administration device package of the fourth embodiment.
Fig. 27 is a view which schematically shows the production process of the transdermal administration device package of the fourth embodiment.
Fig. 28 is a view which schematically shows a process in which the transdermal administration device fixed to a protective sheet is formed in the production process of the transdermal administration device package of the fourth embodiment.
Fig. 29 is a view which schematically shows a process in which the transdermal administration device fixed to the protective sheet is covered with a casing sheet and the protective sheet and the casing sheet are punched to form the transdermal administration device package in the production process of the transdermal administration device package of the fourth embodiment.
Fig. 30 includes (A) and (B) which are scanning electron microscope images of the transdermal administration device of Example 4.
Fig. 31 includes (A) to (D) which are microscopic images of the transdermal administration device of Example 5.

### [Description of Embodiments]

### First Embodiment

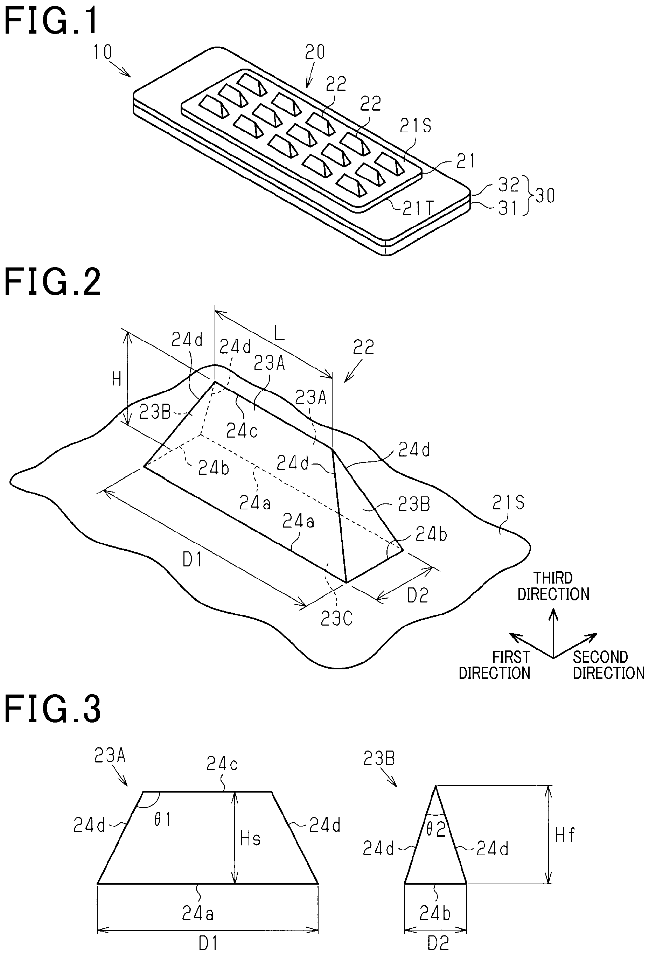

With reference to Figs. 1 to 7, an embodiment of a transdermal administration device is described as a first embodiment.

### [Overall Configuration of Transdermal Administration Device]

With reference to Fig. 1, an overall configuration of a transdermal administration device will be described.

As shown in Fig. 1, a transdermal administration device 10 includes an administration section 20 and an adhesive sheet 30.

The administration section 20 includes a plate-shaped substrate 21 and a plurality of projections 22 which protrude from the substrate 21. The substrate 21 has a first surface 21S on which the projections 22 are formed and a second surface 21T which is a surface opposite from the first surface 21S. The first surface 21S supports base ends of the projections 22.

The adhesive sheet 30 includes a base sheet 31 and an adhesive layer 32 which covers one of two surfaces of the base sheet 31. The second surface 21T of the substrate 21 is bonded to a portion of the adhesive surface of the adhesive layer 32.

### [Configuration of Projection]

With reference to Figs. 2 and 3, a detailed structure of a projection will be described.

As shown in Fig. 2, a projection 22 is a filled solid made up of two main lateral faces 23A which are trapezoidal flat surfaces, two auxiliary lateral faces 23B which are triangular flat surfaces, and one base 23C which is a rectangular flat surface.

The base 23C is a defined surface located inside the first surface 21S of the substrate 21. The base 23C is defined by four sides, of which two long sides 24a extend in a first direction and two short sides 24b, which are shorter than the long side 24a, extend in a second direction. The first direction and the second direction are directions parallel to the first surface 21S, and the first direction and the second direction are perpendicular to each other. Further, a direction perpendicular to the first surface 21S, that is, a direction perpendicular to the first direction and the second direction is a third direction.

The two main lateral faces 23A have identical isosceles trapezoid shapes, and one of the two main lateral faces 23A intersects the base 23C at a long side 24a, and the other of the two main lateral faces 23A intersects the base 23C at the other long side 24a. The main lateral faces 23A are each inclined relative to the third direction such that the two main lateral faces 23A intersect each other at their top edges 24c, which are opposite sides parallel to the corresponding long sides 24a of the main lateral faces 23A. That is, the two main lateral faces 23A have the common top edge 24c and separate lateral edges 24d. The top edge 24c has two ends, one of which is referred to as a first end, and the other is referred to as a second end. Two lateral edges 24d extend from the first end of the top edge 24c, which are referred to as first lateral edges, and another two lateral edges 24d extend from the second end of the top edge 24c, which are referred to as second lateral edges.

The two auxiliary lateral faces 23B have the identical isosceles triangular shapes. One of the auxiliary lateral faces 23B, which is a first auxiliary lateral face, intersects the base 23C at its short side 24b, and the other of the auxiliary lateral faces 23B, which is a second auxiliary lateral face, intersects the base 23C at its short side 24b. The auxiliary lateral faces 23B are each inclined relative to the third direction. Each auxiliary lateral face 23B intersects one of the two main lateral faces 23A at one of the two lateral edges 24d, which are equal sides of the isosceles triangle, and intersects the other of the two main lateral faces 23A at the other of the two lateral edges 24d. That is, the auxiliary lateral face 23B and each of the two main lateral faces 23A have the common lateral edges 24d. In other words, the first auxiliary lateral face and each of the two main lateral faces 23A have the common first lateral edge, and the second auxiliary lateral face and each of the two main lateral faces 23A have the common second lateral edge. Further, two main lateral faces 23A and one auxiliary lateral face 23B interposed between the two main lateral faces 23A form a corner. That is, the projection 22 has a corner formed by the two main lateral faces 23A and the first auxiliary lateral face, and another corner formed by the two main lateral faces 23A and the second auxiliary lateral face.

In the projection 22, the top edge 24c is a tip. That is, the tip of the projection 22 is formed in a linear shape extending along the first direction.

Thus, the projection 22 has a blade shape extending along the first direction.

The projection 22 has a height H which extends from the first surface 21S of the substrate 21 to the tip of the projection 22 in the third direction. The height H is preferably in the range of 10 µm or more and 1000 µm or less, and is determined within this range depending on the depth required for a hole to be created by the projection 22 into the administration target, that is, a passage through which a drug is administered into the skin.

When the administration target is the human skin and the depth of the hole is designed to be in the stratum corneum, the length H is preferably in the range of 10 µm or more and 300 µm or less, more preferably in the range of 30 µm or more and 200 µm or less. When the depth of the hole is designed to penetrate through the stratum corneum and not to reach the nerve, the height H is preferably in the range of 200 µm or more and 700 µm or less, more preferably in the range of 200 µm or more and 500 µm or less, and further more preferably in the range of 200 µm or more and 300 µm or less. When the depth of the hole is designed to reach the dermis, the height H is preferably in the range of 200 µm or more and 500 µm or less. When the depth of the hole is designed to reach the epidermis, the height H is preferably in the range of 200 µm or more and 300 µm or less.

The projection 22 has a width D1 in the first direction, which is a maximum length of the projection 22 in the first direction. Further, the projection 22 has a width D2 in the second direction, which is a maximum length of the projection 22 in the second direction. That is, the width D1 is a length of the long side 24a and the width D2 is a length of the short side 24b, and the width D1 is larger than the width D2. Specifically, the width D1 is preferably in the range of 200 µm or more and 2000 µm or less, and the width D2 is preferably in the range of 1 µm or more and 1000 µm or less.

The projection 22 has a length L of the tip, which is a length of a linear portion of the projection 22 farthest from the first surface 21S of the substrate 21, that is, a length of the top edge 24c. The length L of the tip is smaller than the width D1 and larger than the width D2. Specifically, the length L of the tip is preferably in the range of 100 µm or more and 1000 µm or less.

With reference to Fig. 3, the shape of the main lateral face 23A and the auxiliary lateral face 23B will be described in detail.

As shown in Fig. 3, the main lateral face 23A has an angle θ1 of a main apex angle made by the top edge 24c, which constitutes the tip of the projection 22, and the lateral edge 24d, which is a side connecting the end of the top edge 24c and the first surface 21S of the substrate 21. The angle θ1 is an obtuse angle which is larger than 90 degrees and smaller than 180 degrees. Specifically, the angle θ1 of the main apex angle is preferably in the range of 110 degrees or more and 150 degrees or less.

The main lateral face 23A has a height Hs which is a height of the isosceles trapezoid, that is, a minimum length from the long side 24a to the top edge 24c. An aspect ratio As of the main lateral face 23A is a ratio of the height Hs to the length of the long side 24a, that is, the width D1 of the projection 22 (As = Hs/D1). The aspect ratio As is preferably smaller than 1, more preferably in the range of 0.05 or more and 0.8 or less.

The auxiliary lateral face 23B has an angle θ2 of an auxiliary apex angle, which is an apex of the isosceles triangle, that is, an angle formed between the two lateral edges 24d. The angle θ2 is an acute angle which is smaller than 90 degrees. Specifically, the angle θ2 of the auxiliary apex angle is preferably in the range of 10 degrees or more and 60 degrees or less.

The auxiliary lateral face 23B has a height Hf which is a height of the isosceles triangle, that is, a minimum length from the short side 24b to the apex of the auxiliary lateral face 23B. An aspect ratio Af of the auxiliary lateral face 23B is a ratio of the height Hf to the length of the short side 24b which is a base side of the isosceles triangle, that is, the width D2 of the projection 22 (As = Hf/D2). The aspect ratio Af is preferably larger than 1, more preferably in the range of 1.2 or more and 4.6 or less.

Further, when the projection 22 having the above configuration is viewed in a direction along the second direction, an angle between the side that constitutes the tip of the projection 22 and the side that connects the tip of the projection 22 and the first surface 21S of the substrate 21 of the projection 22 is an obtuse angle larger than the angle θ1. In addition, when the projection 22 is viewed in a direction along the first direction, an angle between two sides that connect the tip of the projection 22 and the first surface 21S of the substrate 21 is preferably an acute angle larger than the angle θ2.

### [Array of Projections]

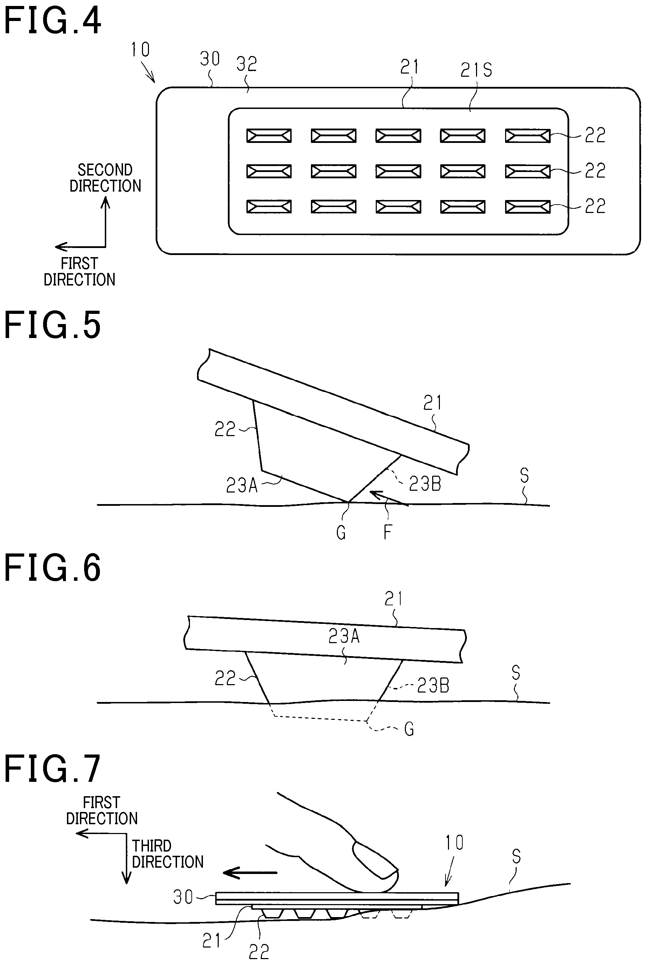

With reference to Fig. 4, a detailed configuration of the transdermal administration device will be described, focusing on the array of projections.

As shown in Fig. 4, when viewed in the direction perpendicular to the first surface 21S of the substrate 21, the substrate 21 has an outer shape having a longer dimension in the first direction than in the second direction. For example, the substrate 21 has a rectangular shape or an ellipse shape which is longer in the first direction than in the second direction.

When viewed in the direction perpendicular to the first surface 21S of the substrate 21, the adhesive sheet 30 has an outer shape larger than the substrate 21 and having a longer dimension in the first direction than in the second direction substrate 21. For example, the adhesive sheet 30 has a rectangular shape or an ellipse shape which is similar to the shape of the substrate 21. When viewed in the direction perpendicular to the first surface 21S of the substrate 21, the adhesive sheet 30 extends outward from the substrate 21 such that the adhesive surface of the adhesive layer 32 is exposed.

In the above configuration, when viewed in the direction perpendicular to the first surface 21S of the substrate 21, an extending direction of the projection 22, an extending direction of the substrate 21, and an extending direction of the adhesive sheet 30 are aligned. In other words, when viewed in the direction perpendicular to the first surface 21S of the substrate 21, an extending direction of the tip of the projection 22, a longitudinal direction of the substrate 21 along which the long side or longer diameter of the substrate 21 extends, and a longitudinal direction of the adhesive sheet 30 along which the long side or longer diameter of the adhesive sheet 30 extends are aligned.

The number of projections 22 is not specifically limited, but is one or more. When the administration section 20 includes a plurality of projections 22, the plurality of projections 22 are arranged with the extending direction of the tips of the projections 22 being aligned as shown in Fig. 4. The plurality of projections 22 may be regularly or irregularly arranged on the first surface 21S of the substrate 21 as long as the extending direction of the tips of the respective projections 22 are aligned with each other and the plurality of projections 22 includes the projections 22 that are disposed at different positions in the extending direction of the substrate 21. In other words, the projections 22 are only required to be positioned such that the extending direction of the tip of the projection 22, the longitudinal direction of the substrate 21, and the longitudinal direction of the adhesive sheet 30 are aligned, and, the projections 22 are located at different positions in the longitudinal direction. For example, in the example shown in Fig. 4, the plurality of projections 22 are arranged in a grid pattern in the first direction and the second direction.

### [Forming Materials of Transdermal Administration Device]

Forming materials of the transdermal administration device 10 in the first embodiment will be described.

The administration section 20 can be made of silicon, metal, ceramic, resin, or a material that dissolves in water contained in the skin. The forming material of the administration section 20 is preferably a biocompatible material. A metal material used as a forming material of the administration section 20 may be stainless steel, titanium, manganese or the like, and a ceramic material used as a forming material of the administration section 20 may be glass, alumina or the like. However, the forming material of the administration section 20 is not limited to these materials. A resin used for forming the administration section 20 may be a medical grade silicone resin, polylactic acid, polyglycolic acid, polycarbonate, polyethylene, polypropylene, epoxy resin, polyamide resin, phenolic resin, polystyrene resin, polycaprolactone, acrylic resin, urethane resin, aromatic polyether ketone, cyclic olefin copolymer or the like. However, the forming material of the administration section 20 is not limited to these materials.

In the configuration in which the administration section 20 is made of a material that dissolves in water contained in the skin, the projection 22 dissolves in the skin after it is pierced into the skin. Examples of a material that dissolves in water contained in the skin, that is, a water soluble material, include a water soluble polymer and disaccharide.

Examples of water soluble polymer include carboxymethyl cellulose (CMC), methylcellulose (MC), hydroxylpropyl cellulose (HPC), hydroxypropyl methylcellulose (HPMC), polyvinyl alcohol (PVA), polyacrylic acid polymer, polyacrylic amide (PAM), polyethylene oxide (PEO), pullulan, alginate, pectin, chitosan, chitosan succinamide, and oligochitosan. Among these materials, chitosan, oligochitosan, chitosan succinamide, carboxymethyl cellulose (CMC), hydroxypropyl cellulose (HPC), and hydroxypropyl methylcellulose (HPMC) are particularly desirable as a material for the projection 22 since they have high biological safety. However, the forming material of the administration section 20 is not limited to these materials.

The disaccharide is preferably trehalose or maltose. In particular, when the projection 22 includes protein as a drug, trehalose serves to protect and stabilize the protein since trehalose, among others, has a crystal structure close to that of water. However, the forming material of the administration section 20 is not limited to these materials.

Further, the projection 22 may also include an additive such as stabilizer in addition to a water soluble polymer and disaccharide.

A drug administered by the administration section 20 may be any kind of drug as long as it works when administered into the skin. A drug may be applied on the surface of the projection 22 and delivered into the skin as the projection 22 creates a hole in the skin. Alternatively, when the projection 22 is made of a soluble material as described above, a drug may be contained inside the projection 22 and delivered into the skin as the projection 22 dissolves. Further, a liquid drug may be applied on the skin before or after the projection 22 is pierced into the skin so that the drug is delivered into the skin through a hole created by the projection 22. Moreover, a drug may be applied by combinations of these techniques. When the projection 22 is made of a soluble material, a water soluble polymer that constitutes the projection 22 may serve as a drug.

A drug may be, for example, various types of proteins, pharmacologically active agents, or cosmetic compositions, and is appropriately selected depending on the purpose.

Examples of a pharmacologically active agent include vaccines such as influenza vaccine, pain relievers for cancer patients, insulin, biologics, gene therapy agents, injections, oral agents, skin application preparations and the like. In transdermal administration using the administration section 20, a drug is administered into a hole created in the skin. Therefore, transdermal administration using the administration section 20 can be applied to not only administration of the pharmacologically active agents used in the conventional transdermal administration, but also administration of pharmacologically active agents that requires hypodermic injection. In particular, since transdermal administration using the administration section 20 does not cause pain by administration, it is suitable for administration of an injection drug such as vaccines for children. Further, since transdermal administration using the administration section 20 does not require oral administration of a drug, it is suitable for administration of an oral drug for children who have difficulty in taking an oral drug.

Cosmetic compositions are compositions for use as cosmetics or beauty products. Examples of a cosmetic composition include humectants, colorants, fragrance, and physiologically active agents exhibiting cosmetic effects such as improvement effect on wrinkles, acne, stretch marks or the like, and improvement effect on hair loss or the like. When an aromatic material is used as a cosmetic composition, a fragrance can be imparted to the administration section 20. Accordingly, the transdermal administration device 10 suitable for use as a beauty product can be obtained.

In addition, the substrate 21 and the projection 22 may be made of a material having the same composition, or materials having different compositions. In the configuration in which the substrate 21 and the projection 22 are made of a material having the same composition, the substrate 21 and the projection 22 can be easily integrally formed. Moreover, when the substrate 21 is made of a soluble material, a drug may also be contained in the substrate 21. As the substrate 21 dissolves in a surface layer of the skin, the drug contained in the substrate 21 is introduced into the surface layer of the skin.

Although materials for the adhesive sheet 30 are not specifically limited, the base sheet 31 is formed of, for example, a resin film made of polyethylene terephthalate or the like, and the adhesive layer 32 is formed of, for example, an epoxy or acrylic adhesive. An adhesive is preferably made of a material having properties suitable for a skin patch, and more preferably, a material that withstands a sterilization process.

### [Effects]

With reference to Figs. 5 to 7, effects of the transdermal administration device 10 will be described while describing the steps of drug administration to the skin by the transdermal administration device 10.

In use of the transdermal administration device 10, the projection 22 is oriented to the skin of administration target, the substrate 21 is pressed against the skin, and the adhesive sheet 30 exposed outside the substrate 21 is affixed to the skin.

Here, it is difficult to place the substrate 21 parallel to the skin surface and press the entire surface of the first surface 21S against the skin at a time, since the substrate 21 and the adhesive sheet 30 have a certain amount of surface area and the surface of the skin of administration target is not a completely flat surface. Usually, edges of the adhesive sheet 30 and the substrate 21 are first pressed against the skin.

As a result, as shown in Fig. 5, the projection 22 is pierced into the skin while the substrate 21 is in an inclined position to a skin surface S. Accordingly, a corner G formed by two main lateral faces 23A and the auxiliary lateral face 23B between the two main lateral faces 23A of the projection 22 is first pierced into the skin. At this time, due to resistance from the skin, the corner G is subject to a force which presses the corner G in a direction away from the skin surface S. In particular, the auxiliary lateral face 23B of the corner G is subject to a reactive force F which presses the corner G in the first direction.

In the first embodiment, since the main apex angle of the main lateral face 23A is an obtuse angle, the corner G has a high strength compared with a case where the main apex angle is a right angle or an acute angle. As a result, the corner G is prevented from being bent or collapsed.

On the other hand, since the auxiliary apex angle of the auxiliary lateral face 23B is an acute angle, the corner G has sharpness compared with a case where the auxiliary apex angle is a right angle or an acute angle. Accordingly, even if the main apex angle is an obtuse angle, the sharpness of the corner G as a whole is prevented from being excessively reduced.

Further, as shown in Fig. 6, once the corner G is pierced into the skin, the tip of the projection 22 adjacent to the corner G is gradually inserted into the skin. The projection 22 is then advanced into the skin to be entirely inserted into the skin. Here, since the main apex angle of the main lateral face 23A is an obtuse angle, a hole which is first incised by the corner G is large compared with a case where the main apex angle is a right angle or an acute angle. Accordingly, the tip of the projection 22 is easily inserted into the skin by using a hole incised by the corner G as a starting point. As a result, the entire projection 22 can be easily inserted into the skin.

Further, as shown in Fig. 7, it is easier for a user of the transdermal administration device 10 to press the substrate 21 against the skin via the adhesive sheet 30 along the longitudinal direction of the adhesive sheet 30 and the substrate 21 than to press the substrate 21 against the skin along other directions.

On the other hand, in piercing of the projection 22 into the skin, the projection 22 needs to be pressed against the skin along the direction in which the tip extends.

In the first embodiment, the extending direction of the tip of the projection 22, the longitudinal direction of the substrate 21, and the longitudinal direction of the adhesive sheet 30 are aligned. Accordingly, the direction in which a user of the transdermal administration device 10 can easily press the substrate 21 against the skin matches the direction in which the projection 22 should be pressed against the skin. Therefore, the projection 22 can be easily pierced into the skin.

For example, in Fig. 7, the substrate 21 is pressed from the edge against the skin with a user's finger pressing the adhesive sheet 30 from the edge along the longitudinal direction of the adhesive sheet 30, that is, the first direction. As a result, the projection 22 is pressed against the skin along the extending direction of the tip, and is pierced into the skin from the corner G as previously shown in Figs. 5 and 6.

In addition, compared with a needle-shaped projection of the conventional microneedles, the projection 22 of the first embodiment can ensure a large volume and a large surface area, and thus a large amount of drug capable of being contained in the projection 22 and a large amount of drug capable of being applied on the surface of the projection 22. Further, compared with the above projection of the conventional microneedles, the projection 22 of the first embodiment can ensure a large area of the base which is defined within the first surface 21S of the substrate 21. As a result, in a production process of the administration section in which an intaglio plate is filled with a material for the administration section, which will be described later, a recess that corresponds to an administration section can be easily filled with the material.

From these points of view, the projection 22 is preferably made of a water soluble material or made of both a water soluble material and a drug from among the above materials. In this configuration, deformation of the projection 22 can be prevented, and furthermore, at least one of the amount of a substance delivered into the skin by the projection 22, that is, the amount of a water soluble material and the amount of a drug can be increased. The conventional projection often has a cone or pyramid shape as described above, which is a conical shape elongated in a direction perpendicular to the first surface 21S of the substrate 21. Since a projection of a conical shape does not have sufficient volume, the amount of substance delivered into the skin by the conical shape projection may be insufficient. In contrast, the projection 22 of the transdermal administration device 10 according to the present embodiment has a large volume for the projection, compared with that of the conical shape projection. Accordingly, the amount of substance delivered into the skin can be increased by the projection.

Although the projection 22 is preferably made of a water soluble material or made of both a water soluble material and a drug, the projection 22 may also include an additive such as stabilizer in addition to these materials.

As described above, the transdermal administration device 10 of the first embodiment can reduce occurrence of pain or fear in a patient by using fine projections similar to the conventional microneedle, and can perform drug administration without using a large device. Furthermore, the projection 22 can be prevented from being deformed. The inventors of the present application have focused on the function of the projection 22 to form a passage for drug administration by piercing the skin, in particular, focused on at what orientation the projection 22 is inserted and advanced into the skin, and arrived at the idea of the shape of the projection 22 in the present embodiment.

As described above, according to the transdermal administration device of the first embodiment, the following effects can be obtained.
(1) Since the angle θ1 of the main apex angle of the main lateral face 23A is an obtuse angle and the angle θ2 of the auxiliary apex angle of the auxiliary lateral face 23B is an acute angle, the sharpness of the corner G formed by the main lateral faces 23A and the auxiliary lateral face 23B viewed in the first direction is prevented from being excessively reduced and the strength of the corner G against an external force in the first direction can be enhanced. Therefore, deformation of the projection 22 can be reduced.
(2) Since the aspect ratio Af of the auxiliary lateral face 23B is larger than 1, the auxiliary lateral face 23B has a sharper shape than the auxiliary lateral face 23B with the aspect ratio Af of not more than 1. As a result, the corner G has higher sharpness, which facilitates piercing of the projection 22.
(3) Since the auxiliary lateral face 23B is a triangular flat surface having an apex made by an end of the top edge 24c, designing of an angle of the main apex angle or the auxiliary apex angle is facilitated, and further, production of the projection 22 is also facilitated.
(4) Since the extending direction of the tip of the projection 22, the extending direction of the substrate 21, and the extending direction of the adhesive sheet 30 are aligned, the direction in which a user of the transdermal administration device 10 can easily press the substrate 21 against the skin matches the direction in which the projection 22 should be pressed against the skin. Therefore, the projection 22 can be easily pierced into the skin.

### (Second Embodiment)

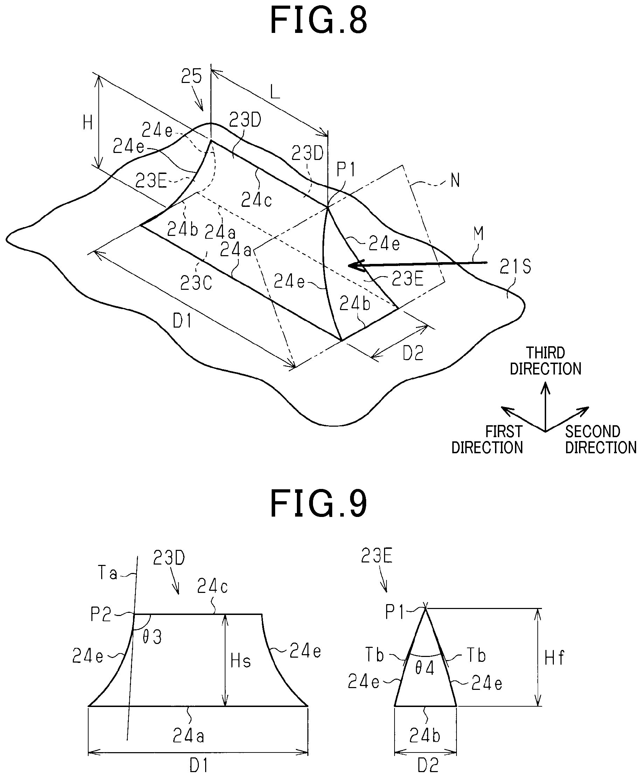

With reference to Figs. 8 and 9, another embodiment of a transdermal administration device is described as a second embodiment. The second embodiment differs from the first embodiment in that the auxiliary lateral face of the projection is a curved surface. The second embodiment will be described focusing on differences from the first embodiment. Configurations the same as those of the first embodiment will be referred to by the same reference numbers, and the description thereof will be omitted.

### [Configuration of Projection]

As shown in Fig. 8, a projection 25 of the second embodiment is a filled solid made up of two main lateral faces 23D which are flat surfaces, two auxiliary lateral faces 23E which are curved surfaces, and one base 23C.

The two main lateral faces 23D have the identical shape, and each main lateral face 23D is surrounded by the long side 24a and the top edge 24c which are parallel to each other, and two curved lateral edges 24e which connect the long side 24a and the top edge 24c. The main lateral faces 23D are each inclined relative to the third direction such that the two main lateral faces 23D intersect each other at their top edges 24c. The top edge 24c constitutes the tip of the projection 25, and tip of the projection 25 is formed in a linear shape extending along the first direction. Further, each main lateral face 23D has an axisymmetric shape to a perpendicular bisector of the long side 24a.

The two auxiliary lateral faces 23E have the identical shape, and each auxiliary lateral face 23E is a curved surface surrounded by the short side 24b and two lateral edges 24e and having a curvature which curves inward to the projection 25. The auxiliary lateral faces 23E are each inclined relative to the third direction. Each auxiliary lateral face 23E intersects one of the two main lateral faces 23D at one of the two lateral edges 24e and intersects the other of the two main lateral faces 23D at the other of the two lateral edges 24e.

That is, the lateral edge 24d of the projection 22 in the first embodiment has a linear shape, while the lateral edge 24e of the projection 25 in the second embodiment has a curved shape. Here, one plane formed by connecting three apexes on the auxiliary lateral face 23E, that is, one plane having an apex P1 which is an intersection between two lateral edges 24e and the short side 24b is defined as a virtual plane N. A direction perpendicular to the virtual plane N is a normal direction M, which is indicated by the arrow in Fig. 8.

The height H, the width D1 in the first direction, the width D2 in the second direction, and the length L of the tip of the projection 25 are preferably in the range described previously for the height H, the width D1 in the first direction, the width D2 in the second direction, and the length L of the tip of the projection 22 of the first embodiment.

With reference to Fig. 9, the shape of the main lateral face 23D and the auxiliary lateral face 23E will be described in detail. In Fig. 9, the auxiliary lateral face 23E is shown projected onto the virtual plane N.

As shown in Fig. 9, the main lateral face 23D has an angle θ3 of a main apex angle made by the top edge 24c, which constitutes the tip of the projection 25, and the lateral edge 24e, which is a side connecting the end of the top edge 24c and the first surface 21S of the substrate 21. The angle θ3 is an obtuse angle which is larger than 90 degrees and smaller than 180 degrees. Specifically, the angle θ3 of the main apex angle is preferably in the range more than 90 degrees and 135 degrees or less.

The main apex angle is an angle formed between a tangent Ta and the top edge 24c. The tangent Ta is a line extends from the apex P2, which is an intersection between the top edge 24c of the main lateral face 23D and the lateral edge 24e, to be tangent to the lateral edge 24e.

The main lateral face 23D has the height Hs which is a minimum length from the long side 24a to the top edge 24c. An aspect ratio As of the main lateral face 23D is a ratio of the height Hs to the width D1 of the projection 22 (As = Hs/D1). The aspect ratio As is preferably smaller than 1, more preferably in the range of 0.05 or more and 0.8 or less.

The auxiliary lateral face 23E has an angle θ4 of an auxiliary apex angle, which is an angle formed between the two lateral edges 24e. The angle θ4 is an acute angle which is smaller than 90 degrees. Specifically, the angle θ4 of the auxiliary apex angle is preferably in the range of 10 degrees or more and 60 degrees or less.

When the auxiliary lateral face 23E is viewed in the normal direction M perpendicular to the virtual plane N, that is, when the auxiliary lateral face 23E is projected onto the virtual plane N, the auxiliary apex angle is an angle formed between each of the lateral edges 24e, that is, an angle formed between two tangents Tb which extend from the apex P1, which is an intersection between the two lateral edges 24e, to be tangent to each of the lateral edges 24e.

The auxiliary lateral face 23E has the height Hf which is a minimum length from the short side 24b when the auxiliary lateral face 23E is projected onto the virtual plane N to the apex P1 of the auxiliary lateral face 23E. An aspect ratio Af of the auxiliary lateral face 23E is a ratio of the height Hf to the width D2 of the projection 22 (Af = Hf/D2). The aspect ratio Af is preferably larger than 1, more preferably in the range of 1.2 or more and 4.6 or less.

When the projection 25 having the above configuration is viewed in a direction along the second direction, an angle between the side that constitutes the tip of the projection 25 and the side that connects the tip of the projection 25 and the first surface 21S of the substrate 21 of the projection 25 is an obtuse angle. Further, when the projection 25 is viewed in a direction along the first direction, an angle between two sides that connect the apex of the projection 25 and the first surface 21S of the substrate 21 is an acute angle.

As with the first embodiment, in the transdermal administration device of the second embodiment, the administration section 20 and the adhesive sheet 30 are disposed such that the extending direction of the projection 25, the extending direction of the substrate 21, and the extending direction of the adhesive sheet 30 are aligned when viewed in the direction perpendicular to the first surface 21S of the substrate 21. The transdermal administration device of the second embodiment is also made of a material described as an example of the forming material of the transdermal administration device in the first embodiment.

### [Effects]

Effects of the transdermal administration device of the second embodiment will be described.

As with the first embodiment, the transdermal administration device of the second embodiment also has the main apex angle of the main lateral face 23D which is an obtuse angle, and the auxiliary apex angle of the auxiliary lateral face 23E which is an acute angle. Accordingly, the sharpness of the corner formed by two main lateral faces 23D and one auxiliary lateral face 23E is prevented from being excessively reduced and the strength of the corner can be enhanced. Therefore, deformation of the projection 22 can be reduced.

Further, in the second embodiment, the auxiliary lateral face 23E is a curved surface. As previously shown in Figs. 5 and 6, once the corner of the projection 25 formed by the two main lateral faces 23D and the one auxiliary lateral face 23E is pierced into the skin, the tip of the projection 25 adjacent to the corner is gradually inserted into the skin. The projection 25 is then advanced into the skin to be inserted into the skin. At this time, the auxiliary lateral face 23E which constitutes the corner is pierced into the target by digging into the target. Accordingly, the projection 25 can be easily pierced into the skin in the configuration in which the auxiliary lateral face 23E is a curved surface having a curvature that curves inward to the projection 25, compared with a case where the auxiliary lateral face 23E is a flat surface.

As described above, according to the transdermal administration device of the second embodiment, the following effects can be obtained in addition to the effect (1), (2), and (4) of the first embodiment.
(5) Since the auxiliary lateral face 23E is a curved surface having a curvature that curves inward to the projection 25, the projection 25 can be further easily pierced into the skin.

### [Modified Examples]

The first and second embodiments can be implemented with modifications as described below.
- In the first and second embodiments, the main lateral faces 23A, 23D, and the auxiliary lateral faces 23B, 23E may have a ridge, groove, or hole. A hole may penetrate through or may not penetrate through the projection 22, 25 and the substrate 21 in the third direction. Further, a groove or hole may be filled with a drug.
- The main lateral faces 23A and 23D may be curved surfaces, and the base 23C may not be a rectangular shape. In other words, the shape of each face is not specifically limited as far as the projection has a shape extending along one direction parallel to the first surface 21S, the main apex angle of each of the two main lateral faces that have the common top edge in the form of a linear tip is an obtuse angle, and the auxiliary apex angle of the auxiliary lateral face that is connected to each of the main lateral faces is an acute angle.

Further, when the main lateral face is a curved surface, virtual planes including both ends of the top edge and both ends of the lateral edges are each defined as a projection surface, and the main apex angle is defined on the basis of an image of the main lateral face projected on the projection surface in the normal direction to the projection surface.

In addition, when the short side has a curved shape, the virtual plane N which includes both ends of the two lateral edges is defined as a projection surface as with the second embodiment, the auxiliary apex angle is defined on the basis of an image of the auxiliary lateral face projected on the projection surface in the normal direction to the projection surface. Further, the aspect ratio Af may be determined by a ratio of the height of the auxiliary lateral face to the length between both ends of the short side of the auxiliary lateral face projected onto the virtual plane N.
- In the above embodiments, the main lateral faces 23A and 23D are described as being axisymmetric to the perpendicular bisector of the long side 24a. However, the main lateral faces 23A and 23D may not necessarily have an axisymmetric shape. That is, in each of the two main lateral faces, an angle formed between the top edge and one of the two lateral edges and an angle formed between the top edge and the other of the two lateral edges may be different from each other. The only requirement for the two main lateral faces is that at least one of the angles formed between the top edge and each of the two lateral edges serves as the main apex angle which is an obtuse angle, and the lateral edge which forms the main apex angle of an obtuse angle on one main lateral face and the lateral edge which forms the main apex angle of an obtuse angle on the other main lateral face forms the auxiliary lateral face, forming the auxiliary apex angle which is an acute angle. That is, the angle formed between the first lateral edge and the top edge on each main lateral face is required to be an obtuse angle, and the angle formed between the two first lateral edges on the first auxiliary lateral face is required to be an acute angle.

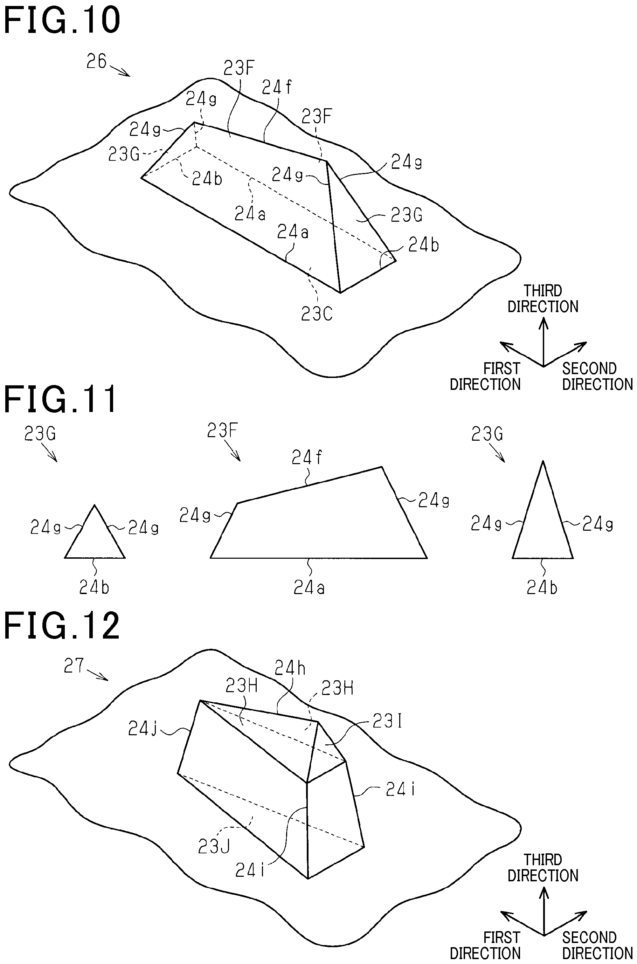

According to this configuration, the effect of the above (1) is achieved by the projection 22, 25 which pierces the skin with the corner formed of the main apex angle which is an obtuse angle and the auxiliary apex angle which is an acute angle. When one of the angles formed by the top edge and each of the two lateral edges on each of the main lateral faces is an acute angle, instructions for the pressing direction of the substrate 21 should be presented to a user, for example by printing on the adhesive sheet 30, so that the user can pierce the projection 22, 25 into the skin with the corner formed of the main apex angle which is an obtuse angle and the auxiliary apex angle which is an acute angle.
- As long as the projection has a shape extending along the first surface 21S of the substrate 21, that is, a shape longer in the first direction than in the third direction, the top edge may extend along a direction other than the first direction. For example, as shown in Fig. 10, the top edge 24f of the projection 26 may be inclined relative to the first surface 21S.

In this case, as shown in Fig. 11, the two lateral edges 24g which form one main lateral face 23F have different lengths. In the main lateral face 23F, the angle formed between the top edge 24f and one of the two lateral edges 24g and the angle formed between the top edge 24f and the other of the two lateral edges 24g are both obtuse angle, but their angles are different from each other. In addition, the two auxiliary lateral faces 23G have different shapes from each other. However, the auxiliary apex angles in each of the auxiliary lateral faces 23G are acute angle.

In other words, the extending direction of the top edge is not necessarily the first direction, and may be any direction different from the third direction. The top edge is required only to be a side having a linear shape and located away from the first surface 21S.
- The projection does not necessarily include the second auxiliary lateral face as long as it has two main lateral faces having the common top edge which is a linear-shaped tip and the separate first lateral edges, and the first auxiliary lateral face having the first lateral edges in common with the respective main lateral faces and forming one corner together with the two main lateral faces. Further, the angle formed between the first lateral edge and the top edge on each main lateral face is required to be an obtuse angle, and the angle formed between the two first lateral edges on the first auxiliary lateral face is required to be an acute angle.

For example, as shown in Fig. 12, the projection 27 includes two main lateral faces 23H having the common top edge 24h, and an auxiliary lateral face 23I having the lateral edges 24i in common with the respective main lateral faces 23H, and does not necessarily have an auxiliary lateral face at a position opposed to the auxiliary lateral face 231 in the first direction.

The main lateral face 23H has a shape made up of trapezoids and triangles connected to the top of the trapezoids, and an inclined angle of the triangular portion in the third direction is larger than an inclined angle of the trapezoidal portion in the third direction. The main apex angle formed by the top edge 24h and the lateral edge 24i on the main lateral face 23H is an obtuse angle. The auxiliary lateral face 231 also has a shape made up of trapezoids and triangles connected to the top of the trapezoids, and an inclined angle of the triangular portion in the third direction is larger than an inclined angle of the trapezoidal portion in the third direction. An auxiliary apex angle of the auxiliary lateral face 231, which is an angle formed between the two lateral edges 24i is an acute angle.

The lateral edge 24i is the first lateral edge that connects the first end of the top edge 24h and the first surface 21S of the substrate 21, and is a polygonal line. The second end of the top edge 24h and the first surface 21S of the substrate 21 are connected to each other by a lateral edge 24j which is one of the second lateral edges. That is, the two main lateral faces 23H have the common lateral edge 24j. Further, a base 23J of the projection 27 has a triangular shape.

In this configuration as well, the effect of the above (1) is achieved by the projection 27 which pierces the skin with the corner formed by the main apex angle which is an obtuse angle and the auxiliary apex angle which is an acute angle. When the transdermal administration device includes the projection 27, instructions for the pressing direction of the substrate 21 should be presented to a user, for example by printing on the adhesive sheet 30, so that the user can pierce the projection 27 into the skin with the above corner.
- The extending direction of the tip of the projection 22, 25 is not necessarily aligned with the longitudinal direction of the substrate 21. Even if the extending direction of the tip of the projection 22, 25 is not aligned with the longitudinal direction of the substrate 21, the direction in which the user of the transdermal administration device can easily press the adhesive sheet 30 against the skin matches the direction in which the projection 22 should be pressed against the skin as long as the extending direction of the tip of the projection 22, 25 is aligned with the longitudinal direction of the adhesive sheet 30. Since the substrate 21 is pressed against the skin along the adhesive sheet 30, the effect similar to the above (4) is achieved.

The extending direction of the tip of the projection 22, 25 is not necessarily aligned with the longitudinal direction of the adhesive sheet 30. For example, a configuration is also possible in which the extending direction of the tip of the projection 22, 25 is not aligned with the longitudinal direction of the adhesive sheet 30, and the extending direction of the tip of the projection 22, 25 is aligned with the longitudinal direction of the substrate 21. In this configuration as well, in a portion of the adhesive sheet 30 to which the substrate 21 is attached, the user of the transdermal administration device can easily press the substrate 21 against the skin in a direction in which the projection 22 should be pressed against the skin. Accordingly, the effect similar to the above (4) is also achieved.

Further, the extending direction of the tip of the projection 22, 25 is not necessarily aligned with either of the longitudinal direction of the substrate 21 and the longitudinal direction of the adhesive sheet 30. In this configuration, for example, instructions for the pressing direction of the substrate 21 should be presented to a user, for example by printing on the adhesive sheet 30, so that the user can pierce the projection 22, 25 into the skin with the corner formed of the main apex angle which is an obtuse angle and the auxiliary apex angle which is an acute angle.
- The transdermal administration device 10 is only required to include at least the administration section 20, and does not necessarily include the adhesive sheet 30.

### (Third Embodiment)

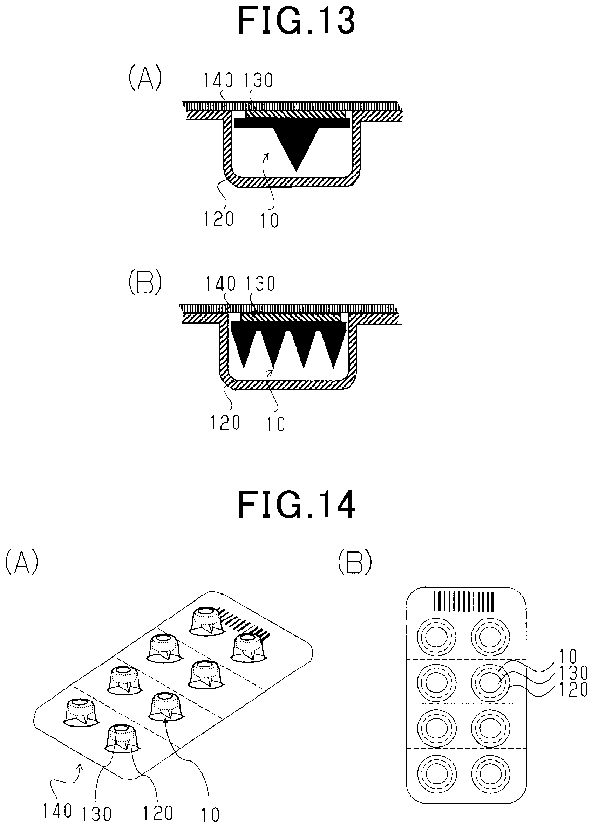

With reference to Figs. 13 and 14, an embodiment of a transdermal administration device package will be described as a third embodiment.

Fig. 13 is a schematic cross sectional view which shows a cross section of an example of a transdermal administration device package in a direction perpendicular to the extending direction of the projection, that is, in the second direction. Fig. 14 is a schematic view which shows a perspective structure and a plan structure of an example of the transdermal administration device package.

As shown in Fig. 13, the transdermal administration device package includes the transdermal administration device 10 according to any one of the first embodiment, the second embodiment, and modifications thereof, a supportive casing 120, an adhesive holder 130, and a protective film 140. Detailed configuration of the support casing 120, the adhesive holder 130, and the protective film 140 will be described in the fourth embodiment.

Fig. 13 shows an example of the transdermal administration device package including the transdermal administration device 10 which is not provided with the adhesive sheet 30. Further, Fig. 13(A) shows an example of the transdermal administration device 10 having one projection, and Fig. 13(B) shows an example of the transdermal administration device 10 having a plurality of projections.

On the surface of substrate of the transdermal administration device 10 opposite from the tip of the projection, that is, on the second surface 21T of the substrate 21 in the configuration in which the transdermal administration device 10 only includes the administration section 20, an adhesive holder 130 is disposed to support the substrate 21. The transdermal administration device 10 is temporarily fixed to the protective film 140 via the adhesive holder 130 attached on the protective film 140. In addition, the transdermal administration device 10 may be temporarily fixed to the support casing 120 instead of the protective film 140.

Fig. 14 shows the transdermal administration device package in which eight transdermal administration devices 10 are fixed to the support casing 120. The eight transdermal administration devices 10 are arranged in two rows and four columns. Fig. 14(A) is a perspective view of the transdermal administration device package, and Fig. 14(B) is a plan view of the transdermal administration device package. As such, the transdermal administration device package may include a plurality of transdermal administration devices 10 and the adhesive holder 130 provided for the respective transdermal administration devices 10.

According to this transdermal administration device package, the transdermal administration device 10 is prevented from being impacted by other objects or being directly touched by the user's hand since the transdermal administration device 10 is packaged. As a result, deformation of the transdermal administration device 10 is prevented.

### (Fourth Embodiment)

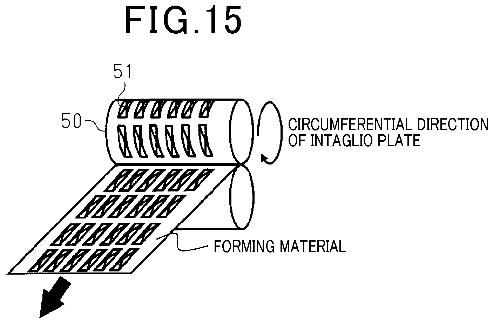

With reference to Figs. 15 to 29, an embodiment of a production method of a transdermal administration device and a production method of a transdermal administration device package are described as a fourth embodiment.

### [Production Method of Transdermal Administration Device]

In the fourth embodiment, an example of a production method of the transdermal administration device 10 according to any one of the first embodiment, the second embodiment, and modifications thereof will be described.

A method for producing the transdermal administration device 10 includes the steps of forming a molded product by filling a recess of an intaglio plate with a forming material of the administration section 20, and removing the molded product from the intaglio plate. The forming material of the administration section 20 may be provided to the intaglio plate singularly or in the form of liquid having the forming material dissolved or dispersed therein.

### <Configuration of Intaglio Plate>

With reference to Fig. 15, an intaglio plate for use in production of the transdermal administration device 10 will be described.

The intaglio plate for use in production of the transdermal administration device includes a recess having a shape in conformity with the shape of the projection of the transdermal administration device 10. The recess is formed to have the longitudinal direction which is an extending direction of the recess when viewed in the direction perpendicular to the surface of the intaglio plate corresponds to a removal proceeding direction, which is a direction in which the molded product made of the forming material of the administration section 20 is removed from the intaglio plate.

Fig. 15 shows that recesses 51 of an intaglio plate 50 which is a roll-shaped mold are filled with the forming material of the administration section 20, and a molded product after being formed is removed from the intaglio plate 50.

As shown in Fig. 15, the plan shape of the respective recesses 51 viewed in the direction perpendicular to the surface of the intaglio plate 50 extends in the removal proceeding direction, that is, the circumferential direction of the intaglio plate 50. In other words, the longitudinal direction of the recess 51 viewed in the direction perpendicular to the surface of the intaglio plate 50 corresponds to the removal proceeding direction.

The above arrangement of the recesses 51 facilitates removal of the molded product from the intaglio plate 50. Accordingly, the precision of shape-transfer from the intaglio plate 50 to the forming material of the administration section 20 is improved.

In addition, the intaglio plate may have a plate-shape as long as the recesses are arranged such that the plan shape of the recesses viewed in the direction perpendicular to the surface of the intaglio plate extends in the removal proceeding direction.

### <Production Method of Intaglio Plate>

As an example of the production method of the above intaglio plate, description will be provided below for the production method of an intaglio plate using a projection having substantially the same shape as that of the projection of the transdermal administration device 10 and a substrate that supports the projection.

<<Production of Original Plate>>

A step of fabricating an original plate is a step of preparing a forming material of the original plate and fabricating an original plate by using a micromachining technique.

The forming material of the original plate is not specifically limited, and is preferably selected considering processing suitability or availability of the material. Examples of the forming material of the original plate include metal materials such as stainless steel (SUS), aluminum and titanium, ceramics such as alumina, aluminum nitride, and machinable ceramics, hard brittle materials such as silicon and glass, and organic materials such as acryl and polyacetal.

The fabrication method of the original plate is not specifically limited, and a known method may be used depending on the shape of the original plate to be fabricated. For example, the original plate may be fabricated by using a micromachining technique or a machine processing technique used for production of semiconductor devices. Specifically, micromachining technique used for fabrication of original plate includes, for example, lithography, wet etching, dry etching, sand blasting, laser processing, and micromachining.

In order to improve the mold releasability of the intaglio plate from the original plate, a surface shape processing or chemical surface modification may be applied on the original plate. Specifically, grinding, boring, or grooving by machine processing, surface treatment and surface modification by using an etching process, or application of a mold release agent may be advantageously used.

The original plate thus obtained is used as a plate for fabrication of the intaglio plate.

### <<Production of Intaglio Plate>>

In production of the intaglio plate, a forming material of the intaglio plate is supplied onto the surface of the original plate. After curing of the forming material of the intaglio plate, the original plate is separated from the forming material to fabricate the intaglio plate having a shape of the original plate reproduced as a recessed shape. The intaglio plate thus fabricated allows for production of a large number of transdermal administration devices 10 from the same intaglio plate. Accordingly, the production cost of the transdermal administration device 10 can be reduced, thereby improving the productivity.

Examples of the forming material of the intaglio plate include inorganic materials such as nickel, silicon, silicon carbide, tantalum, glassy carbon, quartz, and silica, and resin compositions such as silicone resin, urethane rubber, norbornene resin, polycarbonate, polyethylene terephthalate, polystyrene, polymethacrylic acid methyl, acryl, and liquid crystal polymer. Of these materials, considering high formability, conformity with the microshapes, and mold releasability, the forming material is preferably silicone resin, nickel, silicon, silicon carbide, tantalum, glassy carbon, quartz and silica, more preferably silicone resin, and particularly preferably silicone resin containing polydimethyl siloxane. For example, silicone resin containing polydimethyl siloxane with a hardener added thereto can be used. Use of the silicone resin having high mold releasability improves the releasability of cured forming material of the intaglio plate, thereby preventing deformation of the intaglio plate when being removed from the mold.

Further, the production method of the intaglio plate is not limited to the above-mentioned methods, and the intaglio plate can be produced by a known shape-transfer technique. For example, an intaglio plate made of nickel may be produced by nickel electroforming.

The production method of the intaglio plate in the above description includes producing an original plate, and producing an intaglio plate from the original plate. However, the intaglio plate may also be produced by directly processing the forming material of the intaglio plate.

### <Detailed Production Method of Transdermal Administration Device>

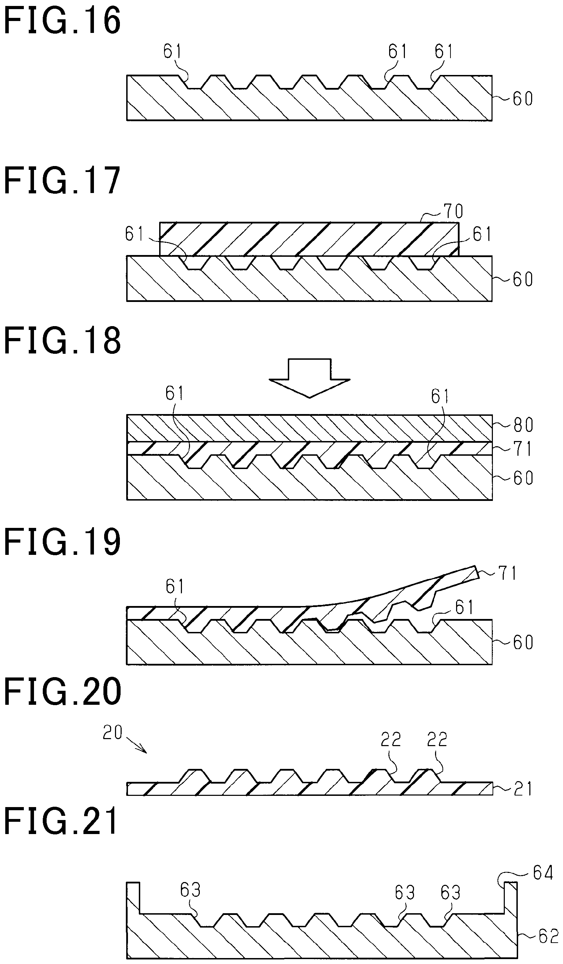

With reference to Figs. 16 to 25, a detailed production method of the transdermal administration device will be described.

When the forming material of the administration section 20 is a thermoplastic resin, the administration section 20 is produced as a molded product by molding the thermally melted forming material by using an intaglio plate. For transfer forming from the intaglio plate to the forming material, a known technique may be used.

When the forming material of the administration section 20 is a material that dissolves in water contained in the skin, such as a water soluble polymer or disaccharide, a liquid material having the forming material dissolved or dispersed in a solvent such as water is first prepared. Then, the liquid material is introduced into the recess of the intaglio plate, and is dried to remove the solvent in the liquid material to thereby form a molded product, which is the administration section 20. For transfer forming from the intaglio plate to the forming material, a known technique may be used.

In the following description, examples of the production method of the transdermal administration device will be described for each of the case which uses a thermoplastic resin and the case which uses a water soluble material as a forming material of the administration section 20.

The production method of the transdermal administration device is only required to include the steps of forming a molded product by filling the recess of the intaglio plate with the forming material of the administration section 20 and removing the molded product from the intaglio plate so that removal is carried out in the extending direction of the recess when viewed in the direction perpendicular to the surface of the intaglio plate, and the details of these steps may be different from the description below.

### <<Production Method of Transdermal Administration Device Using Resin>>

With reference to Figs. 16 to 20, a production method of the transdermal administration device which uses a thermoplastic resin as a forming material of the administration section 20 will be described.

As shown in Fig. 16, an intaglio plate 60 fabricated by the above production method is prepared. The intaglio plate 60 includes a recess 61 having a shape in conformity with the shape of the projection of the administration section 20 of the production target. When the intaglio plate 60 includes a plurality of recesses 61, the plurality of recesses 61 includes the recesses 61 arranged in the extending direction of the recess 61. Further, the intaglio plate is not limited to the plate-shaped intaglio plate 60 shown in Fig. 16, but may be a roll-shaped intaglio plate shown in Fig. 15.

As shown in Fig. 17, a resin material 70 which is a thermoplastic resin is then disposed on the surface of the intaglio plate 60 as a forming material of the administration section 20. The resin material 70 may be supplied onto the intaglio plate 60 in a heated state.

As shown in Fig. 18, the resin material 70 on the intaglio plate 60 is then heated and melted. The resin material 70 is thermally pressed so that the recess 61 is filled with the resin material 70. As a result, a molded product 71 is formed. Although Fig. 18 shows an example of press operation by using a plate-shaped press material 80, press operation may be performed by using a roll-shaped press material. The arrow in Fig. 18 shows a press direction.

As shown in Fig. 19, the molded product 71 is then removed from the intaglio plate 60. Here, the direction in which the molded product 71 made of the forming material of the administration section 20 is removed from the intaglio plate 60 corresponds to the extending direction of the recess 61 viewed in the direction perpendicular to the intaglio plate 60.

As shown in Fig. 20, the removed molded product 71 is the administration section 20. Thus, the administration section 20 is formed. In addition, when a resin is used for the forming material of the administration section 20, the administration section 20 may be produced not only by thermal compression molding as described in Figs. 16 to 20, but also by injection molding or the like.

The obtained administration section 20 is provided as the transdermal administration device 10 by itself or together with other member bonded thereto such as the adhesive sheet 30. The administration section 20 and the adhesive sheet 30 can be bonded to each other by a conventional technique. Further, the molded product 71 and the adhesive sheet 30 may be bonded after the molded product 71 is formed on the intaglio plate 60, and then removed from the intaglio plate 60 to obtain a structure made up of the administration section 20 and the adhesive sheet 30. Moreover, the outer shape of the molded product 71 or the combination of the molded product 71 and the adhesive sheet 30 or the like may be adjusted as necessary by cutting the outer portion of the substrate 21 by punching or the like using a Thomson blade or the like.

### <<Production Method of Transdermal Administration Device Using Water Soluble Material>>

With reference to Figs. 21 to 25, a production method of the transdermal administration device which uses a material that dissolves in water contained in the skin as a forming material of the administration section 20 will be described.

As shown in Fig. 21, an intaglio plate 62 fabricated by the above production method is prepared. The intaglio plate 62 includes a recess 63 having a shape in conformity with the shape of the projection of the administration section 20 of the production target, and a recess 64 having an outer shape in conformity with the outer shape of the substrate of the administration section 20 of the production target. The recess 63 communicates with the recess 64, and the recess 63 is located closer to the bottom of the intaglio plate 62 than the recess 64 is. When the intaglio plate 62 includes a plurality of recesses 63, the plurality of recesses 63 includes the recesses 63 arranged in the extending direction of the recess 63. Further, the intaglio plate is not limited to the plate-shaped intaglio plate 62 shown in Fig. 21, but may be a roll-shaped intaglio plate shown in Fig. 15.

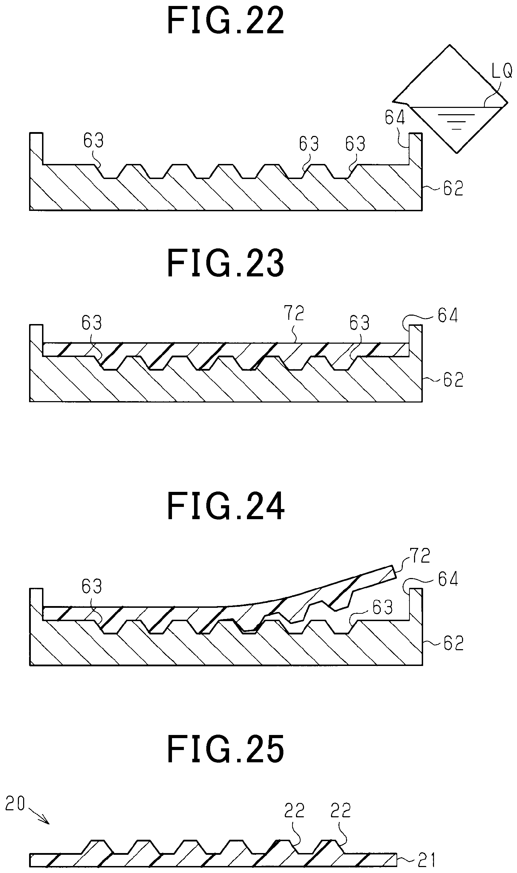

As shown in Fig. 22, a liquid material LQ having the forming material of the administration section 20 dissolved or dispersed in a solvent is prepared, and the liquid material LQ is filled into the recesses 63 and 64 of the intaglio plate 62. The flowability of the liquid material LQ is preferably adjusted to an extent such that the liquid material LQ is smoothly filled into the recesses 63 and 64 by adjusting the amount of the solvent or the like as appropriate. The liquid material LQ can be supplied to the intaglio plate 62 by methods such as spin coating, use of dispenser, casting, and ink jetting. Supplying of the liquid material LQ to the intaglio plate 62 may be performed under normal pressure, but preferably under reduced pressure or vacuum in order to smoothly supply the liquid material LQ into the recesses 63 and 64. Preferably, the supply amount of the liquid material LQ to the intaglio plate 62 is an extent that the liquid material LQ covers at least the entire recess 63. In filling of the liquid material LQ, the liquid material LQ may be pressurized toward the bottom of the intaglio plate 62 so as to facilitate filling of the liquid material LQ into the recesses 63 and 64.

Further, in the case where the compositions of the projection and the substrate are different from each other or the compositions of the tip and the base of the projection are different from each other in the administration section 20 which is the production target, liquid materials which contain forming materials of different compositions may be sequentially filled into the recesses 63 and 64.

As shown in Fig. 23, the liquid material LQ filled in the recesses 63 and 64 of the intaglio plate 62 is dried to remove the solvent. As a result, the molded product 72 is formed.

As shown in Fig. 24, the molded product 72 is then removed from the intaglio plate 62. Here, the direction in which the molded product 72 made of the forming material of the administration section 20 is removed from the intaglio plate 62 corresponds to the extending direction of the recess 63 viewed in the direction perpendicular to the intaglio plate 62.

As shown in Fig. 25, the removed molded product 72 is the administration section 20. Thus, the administration section 20 is formed. The obtained administration section 20 is provided as the transdermal administration device 10 by itself or together with other member bonded thereto such as the adhesive sheet 30. The administration section 20 and the adhesive sheet 30 can be bonded to each other by a conventional technique. Further, the molded product 72 and the adhesive sheet 30 may be bonded after the molded product 72 is formed on the intaglio plate 62, and then removed from the intaglio plate 62 to obtain a structure made up of the administration section 20 and the adhesive sheet 30. Moreover, the outer shape of the molded product 72 or the combination of the molded product 72 and the adhesive sheet 30 or the like may be adjusted as necessary by cutting the outer portion of the substrate 21 by punching or the like using a Thomson blade or the like.

The molded product of the administration section 20 may also be formed by applying pressure onto a sheet made of the forming material of the substrate 21 which is placed on the forming material of the projection filled in the recess 63 of the intaglio plate 62.

### <Advantageous Effect>

Advantageous effects of the production method of the transdermal administration device of the present embodiment will be described.

The conventional production methods of a microneedle which has been proposed include fabricating an original plate of a projection by cutting work, producing an intaglio plate having an inverted pattern of bumps and dents of the original plate, and manufacturing an administration section having a projection made of resin by transfer molding using the intaglio plate. However, these production methods may have a phenomenon, so-called torare in Japanese, that the resin is adhered to the intaglio plate during removal of the molded product from the intaglio plate. If this phenomenon occurs, the precision of shape-transfer from the intaglio plate to the removed molded product is reduced.

Moreover, even if a material other than a thermoplastic resin is used as a forming material of the administration section 20, the forming material may be partially adhered to the intaglio plate during removal of the molded product from the intaglio plate, which may decrease the precision of shape-transfer from the intaglio plate to the removed molded product.

In contrast, according to the production method of the transdermal administration device of the present embodiment, the projection which is the production target has a shape extending along the first surface 21S of the substrate 21, and thus the recess of the intaglio plate for producing the projection has a shape extending in one direction when viewed in the direction perpendicular to the surface of the intaglio plate. Since the molded product is removed from the intaglio plate in the extending direction of the recess when viewed in the direction perpendicular to the surface of the intaglio plate, the molded product is easily removed from the intaglio plate compared with the case where the molded product is removed from the intaglio plate in the direction different from the extending direction of the recess such as that extending perpendicular to the extending direction of the recess. As a result, the forming material is prevented from being partially adhered to the intaglio plate during removal of the molded product. Accordingly, the precision of shape-transfer from the intaglio plate to the removed molded product is improved.

### [Production Method of Transdermal Administration Device Package]

Next, as an example of the production method of the transdermal administration device package of the third embodiment, a production method of the transdermal administration device package having a configuration in which the transdermal administration device 10 is temporarily fixed to the protective film 140 will be described.

This production method of the transdermal administration device package includes the above steps of fabricating the transdermal administration device 10, and the steps of bonding a protective sheet having easy adhesiveness to a surface of the substrate of the transdermal administration device 10 opposite from the tip of the projection via a detachable adhesive material, bonding a casing sheet to the protective sheet so as to cover the tip of the projection on the transdermal administration device 10 and house the transdermal administration device 10, and cutting the protective sheet and the casing sheet bonded to each other.

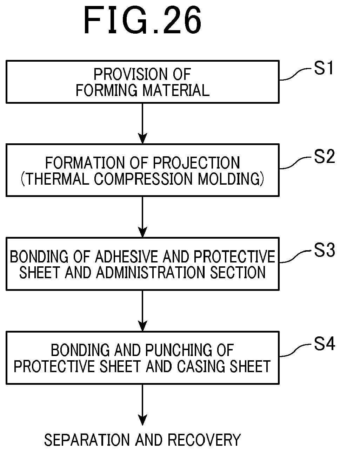

Fig. 26 is a flow chart which shows a production process of the transdermal administration device package when the administration section 20 of the transdermal administration device 10 is made of a thermoplastic resin. Step S1 shown in Fig. 26 is a step of providing a thermoplastic resin which is the forming material of the administration section 20 onto the intaglio plate. Step S2 shown in Fig. 26 is a step of forming a molded product by thermal compression molding by filling the recess of the intaglio plate with the resin and removing the molded product from the intaglio plate to obtain the administration section 20.

That is, the transdermal administration device 10 is fabricated by Steps S1 and S2. Subsequent to Step S2, the protective sheet is bonded to the transdermal administration device 10 via the adhesive material in Step S3. Subsequent to Step S4, the protective sheet and the casing sheet are punched out after the casing sheet and the protective sheet are bonded to each other.

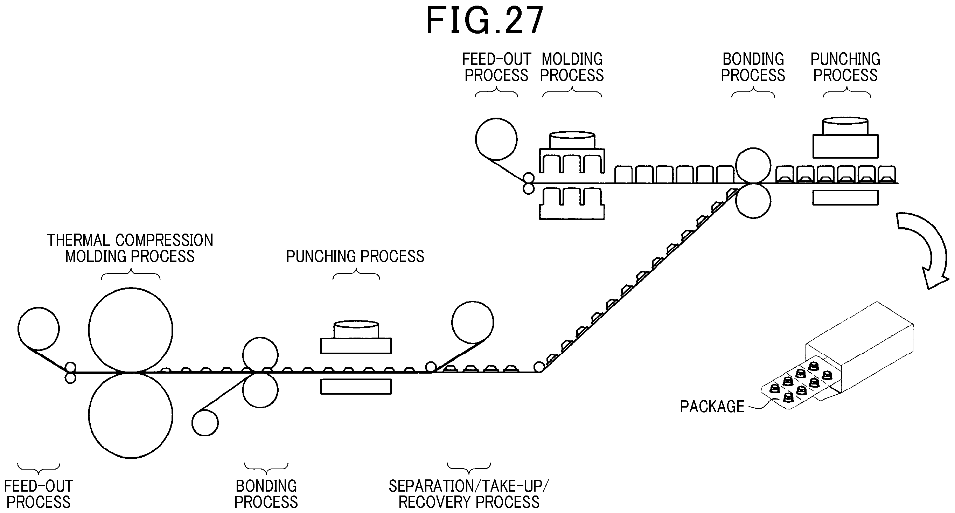

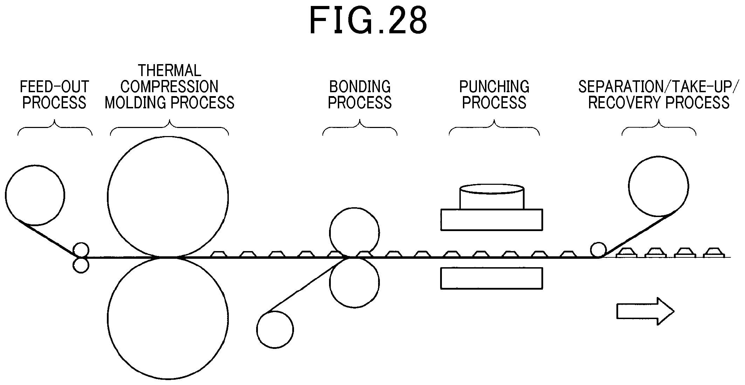

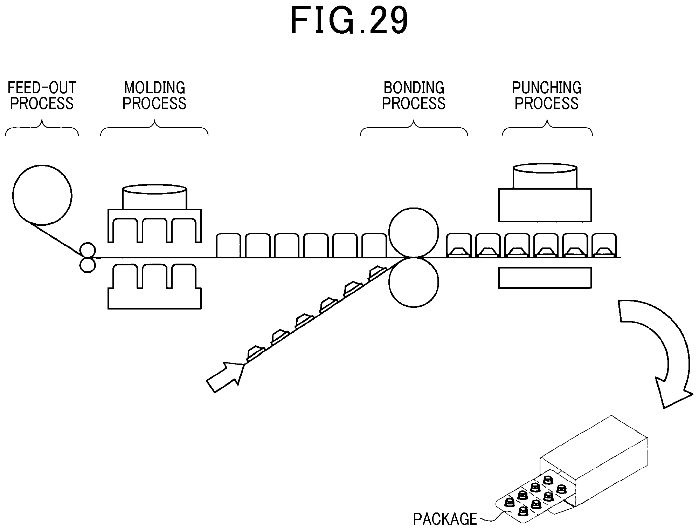

Fig. 27 is a schematic view which shows a production process of the transdermal administration device package. Fig. 28 is a schematic view which shows part of the process shown in Fig. 27 by which the transdermal administration device 10 fixed to the protective sheet is produced. Fig. 29 is a schematic view which shows part of the process shown in Fig. 27 by which the transdermal administration device 10 fixed to the protective sheet is packed with the casing sheet and the protective sheet and the casing sheet are punched out to produce the transdermal administration device package.

In a feeding-out process shown in Fig. 28, a sheet made of resin which is the forming material of the administration section 20 is fed out. In the thermal compression molding process, the resin sheet is heated and pressed against the intaglio plate to produce the projection. Subsequently, in the bonding process, the protective sheet is bonded via the adhesive material to a surface of the resin sheet opposite from the surface on which the projection is formed. Subsequently, in a punching process, the resin sheet and the adhesive material are punched out to produce the administration section 20 and the adhesive holder 130 on the protective sheet. In a separation/take-up/recovery process, the part remaining after the resin sheet and the adhesive material are punched out, that is, the part other than the administration section 20 and the adhesive holder 130 located between the administration section 20 and the protective sheet is separated from the protective sheet, taken up and recovered. As a result, the transdermal administration device 10 fixed to the protective sheet via the adhesive holder 130 is produced.

In the feeding-out process shown in Fig. 29, a sheet made of resin which is the forming material of the support casing 120 is fed out. In the molding process, the resin sheet is molded into a shape of the array of the plurality of recesses of the support casing 120. As a result, a casing sheet is formed. Subsequently, in the bonding process, the casing sheet and the protective sheet are bonded to each other so that the transdermal administration device 10 fixed to the protective sheet is covered by the recess. Subsequently, in the punching process, the casing sheet and the protective sheet are punched out to produce the transdermal administration device package.

The transdermal administration device package thus produced includes, as shown in Fig. 13, the transdermal administration device 10, the support casing 120 formed by punching the casing sheet, the protective film 140 formed by punching the protective sheet, and the adhesive holder 130 disposed between the transdermal administration device 10 and the protective film 140. A space in the transdermal administration device package that houses the transdermal administration device 10 is sealed by the protective film 140 which covers an opening of the support casing 120.

Detailed configuration of the members of the transdermal administration device package will be described.

### <<Adhesive Holder>>

The adhesive holder 130 is made of a material having adhesiveness at least capable of holding the transdermal administration device 10, and may be designed as appropriate depending on the specification of the transdermal administration device 10. For example, the adhesive holder 130 may be formed by a resin having viscoelasticity, an adhesive or the like.

Further, the adhesive holder 130 is preferably detachable to an adhesion target, and is preferably made of a gel polymer. The adhesive holder 130 made of a gel polymer has high adhesiveness, and can be repeatedly attached and removed from the adhesion target. Moreover, the adhesive holder 130 made of a gel polymer can be repeatedly used since the adhesiveness can be restored by cleansing with distilled water, purified water, ethyl alcohol or the like. Specifically, examples of gel polymer include silicone gel, urethane gel and the like.

### <<Support Casing>>

The support casing 120 can be formed by thermal pressing. For example, as described above, a number of recesses, each having a size capable of housing the transdermal administration device 10, are formed by vacuum molding, vacuum pressure molding or the like so as to be arrayed in a matrix on a sheet material having an elongated strip shape. For example, as shown in Fig. 14, the sheet material is cut off into eight recesses arranged in two rows and four columns to form the support casing 120.

The shape of the recess can be designed as appropriate depending on the shape of the transdermal administration device 10, and may be, for example, a circle, ellipse, oblong circle, or a rectangle in plan view. Further, the number of recesses included in one support casing 120 may be arbitrarily determined depending on the number of the transdermal administration devices 10 housed in the support casing 120.

The sheet material used for formation of the support casing 120 may be a sheet material made of a known synthesized resin material such as polyvinyl chloride, polyethylene terephthalate, polypropylene, polyethylene, nylon, or ethylene-vinyl alcohol copolymer resin. Further, the sheet material is preferably a transparent synthesized resin material since it has high gas barrier properties and transparency which allows for visual inspection of the transdermal administration device 10 from the outside of the support casing 120 to easily check a foreign substance in the recess. Specifically, the forming material of the sheet material is preferably an ethylene-vinyl alcohol copolymer.

### <<Protective Film>>

The transdermal administration device 10 is fixed to the protective film 140 by positioning the adhesive holder 130 on one surface of the protective film 140 so that the adhesive holder 130 holds the transdermal administration device 10. Since the transdermal administration device 10 is prevented from being moved in the recess of the support casing 120 during transportation of the transdermal administration device package or the like, the transdermal administration device 10 can be advantageously stored or transported. In particular, when the plurality of transdermal administration devices 10 are housed in one support casing 120, collision between each of the transdermal administration devices 10 can be reduced.

The support casing 120 can be sealed with the protective film 140 by using a heat sealer. That is, the protective sheet which is the protective film 140 is disposed to cover the opening of the support casing 120, and is thermally shrunk by applying heat to the protective sheet to thereby seal the recess in which the transdermal administration device 10 is housed. Sealing of the recess can prevent degeneration of the transdermal administration device 10 due to environmental change.

The protective film 140 is preferably a resin film that has strong adhesiveness to the support casing 120 when being bonded to the support casing 120, and easy openness when being peeled from the support casing 120 to open the transdermal administration device package.

In use of the transdermal administration device package, the user peels the protective film 140 from the support casing 120 and takes out the transdermal administration device 10 which is detachably attached to the protective film 140. Then, the user pierces the projection of the transdermal administration device 10 into the skin. Since the transdermal administration device 10 is packaged, it is prevented from being impacted by other objects or being directly touched by the user's hand. As a result, deformation of the transdermal administration device 10 is prevented.

Further, the transdermal administration device 10 housed in the transdermal administration device package is preferably sterilized. For example, the transdermal administration device 10 can be sterilized by dried heated air, hydrogen peroxide gas, ethylene oxide gas, electron beam radiation, or γ beam radiation. Of these techniques, use of low energy electron beam is widely adopted since the method can be conducted at low temperature and does not leave a residue in the sterilized object, and the handling is safe and easy. When the drug is applied on the transdermal administration device 10, high temperature processing cannot be performed. In addition, especially when the drug is filled in the groove or hole formed on the projection, sterilization must be performed not only on the outer surface of the projection but also on the inner area where the drug is filled. Accordingly, sterilization by γ beam radiation is preferably performed. As described above, sterilization performed on the transdermal administration device 10 can maintain the inside of the support casing 120 of the transdermal administration device package to have an aseptic state over a long period of time.

### [Modified Examples]

The fourth embodiment can be implemented with modifications as described below.
- The transdermal administration device 10 included in the transdermal administration device package may not necessarily be the transdermal administration device 10 produced by the production method of the transdermal administration device according to the fourth embodiment, but may be the transdermal administration device 10 according to any one of the first embodiment, the second embodiment, and modifications thereof. Since the transdermal administration device 10 is packaged as the transdermal administration device package, the transdermal administration device 10 is prevented from being in contact with water, oxygen, carbon dioxide, odors or the like. Accordingly, the transdermal administration device 10 storing a drug which should not be in contact with these gases can be advantageously stored.
- The transdermal administration device 10 according to any one of the first embodiment, the second embodiment, and modifications thereof may be produced by a method different from that of the fourth embodiment. For example, when the molded product is removed from the intaglio plate, the molded product may be removed in the direction different from the extending direction of the recess.

Moreover, the administration section 20 can be produced by various known techniques depending on the forming material of the administration section 20. The administration section 20 may also be produced without using the intaglio plate. For example, when the administration section 20 is made of resin, the administration section 20 can be produced by molding techniques such as injection molding, extrusion molding, imprinting, hot embossing, and casting. Further, the administration section 20 can also be produced by micromachining techniques such as lithography, wet etching, dry etching, sand blasting, and laser processing. In addition, the original plate may be produced by these techniques.

### (Examples)

The above transdermal administration device will be described by using specific examples.

### [Example 1]

### <Fabrication of Intaglio Plate>

The original plate of the administration section was produced from an acrylic plate by micromachining. The projection had the same shape as that of the projection 22 of the first embodiment, and the auxiliary lateral face is a flat surface. The height H of the projection was approximately 500 µm, the width D1 of the projection was approximately 770 µm, and the width D2 of the projection was approximately 280 µm, the length L of the tip was approximately 500 µm, the height Hs of the main lateral face was approximately 520 µm, the height Hf of the auxiliary lateral face was approximately 520 µm, the angle θ1 of the main apex angle was approximately 105 degrees, and the angle θ2 of the auxiliary apex angle was approximately 30 degrees. The 36 projections were arrayed on the substrate in a matrix of six rows and six columns with a pitch of 1 mm.

Then, a thermosetting silicone resin was applied on the surface of the projection of the original plate formed of an acrylic plate, and was removed after being thermally cured to obtain the intaglio plate made of silicone. The recesses which correspond to the 36 projections were formed on the intaglio plate.

### <Fabrication of Transdermal Administration Device>

The liquid material containing the forming material of the administration section, which was 0.1% Evans Blue/5% chitosan succinamide aqueous solution, was filled into the intaglio plate. The intaglio plate was heated at 90°C for 10 minutes so as to dry and solidify the liquid material. The solidified molded product was punched into a circle shape, and removed from the intaglio plate to obtain the administration section.

An adhesive surface of an adhesive sheet which is larger than the substrate was bonded to the second surface of the substrate of the obtained administration section to obtain the transdermal administration device of Example 1.

### [Example 2]

The original plate of the administration section was produced from an aluminum plate by micromachining. The projection had the same shape as that of the projection 25 of the second embodiment, and the auxiliary lateral face is a curved surface. The height H of the projection was approximately 500 µm, the width D1 of the projection was approximately 1500 µm, and the width D2 of the projection was approximately 340 µm, the length L of the tip was approximately 500 µm, the height Hs of the main lateral face was approximately 530 µm, the height Hf of the auxiliary lateral face was approximately 707 µm, the angle θ3 of the main apex angle was approximately 135 degrees, and the angle θ4 of the auxiliary apex angle was approximately 27 degrees. The 36 projections were arrayed on the substrate in a matrix of six rows and six columns with a pitch of 1 mm.

Subsequently, the intaglio plate was fabricated by the same procedure as that of Example 1, the liquid material was filled into the intaglio plate to fabricate the administration section, and the adhesive sheet was bonded to the administration section to obtain the transdermal administration device of Example 2.

### [Example 3]

The liquid material containing the forming material of the administration section, which was 2% dextran/10% glycine/10% trehalose aqueous solution, was filled into the intaglio plate obtained in Example 2 by an ink jet method. The liquid material of 100nl was filled into the respective recesses which correspond to the projections, and then, chitosan succinamide 5% aqueous solution was further filled into the recesses. The dextran was fluorescent-labeled dextran. Thereafter, the intaglio plate was heated at 90°C for 10 minutes so as to dry and solidify the liquid material. The solidified molded product was punched into a circle shape, and removed from the intaglio plate to obtain the administration section having the fluorescent substance concentrated at the tip end of the projection.

The adhesive sheet was bonded to the obtained administration section by the same procedure as that of Example 1 to obtain the transdermal administration device of Example 3.

### [Verification]

The transdermal administration device of Examples 1 to 3 was observed by using a stereoscopic microscope. As a result of observation, it was found that the formation rate of the projections, that is, the rate of the projections of the same shape as those of the original plate were formed on the administration section to the total number of the projections on the original plate was 100% for each of Examples 1 to 3.

The transdermal administration device of Examples 1 to 3 was applied to a mouse. In Example 3, a skin slice sample of the mouse to which the transdermal administration device was applied was prepared, and was observed by using a fluorescence microscope.

The transdermal administration device was applied to the mouse by pressing the projection against the skin along the extending direction of the projection.

In the skin of mouse, blue coloring was observed at a position where the transdermal administration device of Examples 1 and 2 was applied. Further, fluorescence emission was observed from the skin slice of the mouse to which the transdermal administration device of Example 3 was applied. The fluorescence emission was observed at a position in the epidermis or deeper than that. Accordingly, it was found that the drug can be transdermally absorbed by using the transdermal administration device.

### [Example 4]

The original plate of the administration section was produced from an aluminum plate by micromachining. The projection had the same shape as that of the projection 25 of the second embodiment, and the auxiliary lateral face is a curved surface. The height H of the projection was approximately 470 µm, the width D1 of the projection was approximately 770 µm, the length L of the tip was approximately 500 µm, the height Hs of the main lateral face was approximately 500 µm, the angle θ3 of the main apex angle was approximately 105 degrees, and an angle θ4 of the auxiliary apex angle was approximately 60 degrees. The 36 projections were arrayed on the substrate in a matrix of six rows and six columns with a pitch of 1 mm.

Then, the intaglio plate was fabricated in the same procedure as that of Example 1. The liquid material containing the forming material of the administration section, which was hydroxypropyl cellulose aqueous solution, was filled into the intaglio plate. The intaglio plate was then heated at 90°C for 10 minutes so as to dry and solidify the liquid material. Subsequently, the solidified molded product was removed from the intaglio plate. Here, the direction in which the molded product is removed from the intaglio plate corresponded to the extending direction of the recess viewed in the direction perpendicular to the intaglio plate.

Thus, the transdermal administration device of Example 4 formed of the administration section was obtained.

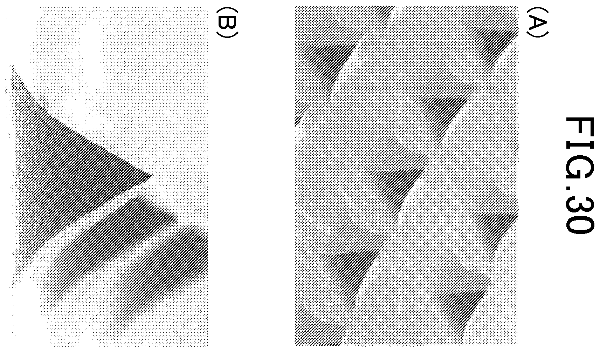

The transdermal administration device of Example 4 was observed by using a scanning electron microscope. The result was that the height H of the projection was 471 µm, the width D1 of the projection was 769 µm, and the angle θ4 of the auxiliary apex angle was 59 degrees. Figs. 30(A) and (B) show the scanning electron microscope images of the transdermal administration device of Example 4.

### [Example 5]

The liquid material containing the forming material of the administration section, which was 0.1% Evans Blue/5% chitosan succinamide aqueous solution, was filled into the recess having a shape in conformity with the shape of the projection of the intaglio plate obtained in Example 4. Then, the intaglio plate was heated at 90°C for 10 minutes so as to dry and solidify the liquid material. Then, the liquid material containing the forming material of the administration section, which was 30% hydroxypropyl cellulose aqueous solution, was filled on the liquid material filled and solidified in the intaglio plate. The intaglio plate was heated at 90°C for 20 minutes so as to dry and solidify the liquid material. Subsequently, the solidified molded product was removed from the intaglio plate. Here, the direction in which the molded product is removed from the intaglio plate corresponded to the extending direction of the recess viewed in the direction perpendicular to the intaglio plate.

Thus, the transdermal administration device of Example 5 formed of the administration section was obtained. In the administration section, an upper layer, that is, the projection is made of chitosan succinamide and Evans Blue, and a lower layer, that is, substrate is made of hydroxypropyl cellulose.

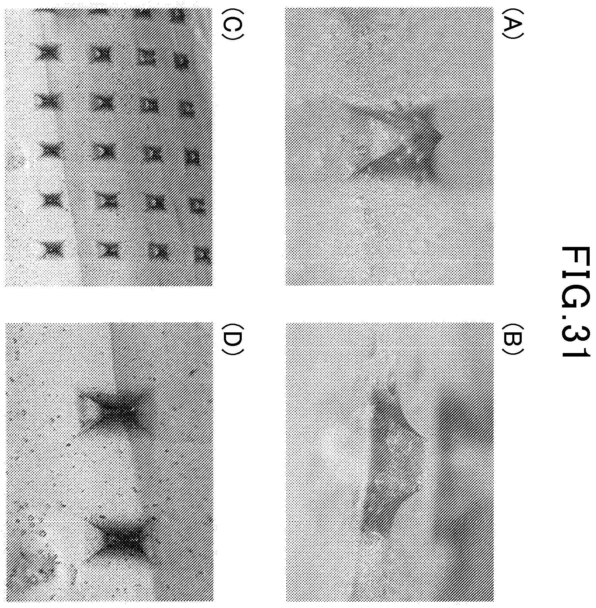

The transdermal administration device of Example 5 was observed by using an optical microscope. It was confirmed that the projection was blue and the substrate was transparent. That is, it was confirmed that the transdermal administration device of Example 5 had a two-layered structure in which the projection was formed of chitosan succinamide and Evans Blue, and the substrate was formed of hydroxypropyl cellulose. Figs. 31(A) to (D) show the microscopic images of the transdermal administration device of Example 5.

### [Example 6]

The original plate which included the projection having the width D1 of approximately 800 µm, the width D2 of approximately 400 µm, and the height H of approximately 700 µm was fabricated, and a roll-shaped intaglio plate was fabricated by inverting the bumps and dents of the original plate. The recess which extends in the circumferential direction of the intaglio plate was formed on the intaglio plate. In other words, when viewed in the direction perpendicular to the surface of the intaglio plate, the long side of the recess, that is, the side having the length of 800 µm which corresponds to the above width D1 extends in the circumferential direction of the intaglio plate, and the short side of the recess, that is, the side having the length of 400 µm which corresponds to the above width D2 extends in the width direction of the intaglio plate.

Polyglycolic acid was used as the forming material of the administration section, and the bumps and dents of the intaglio plate were inverted by thermal compression molding to thereby fabricate the transdermal administration device. As a result, the plurality of projections were formed on the transdermal administration device, and 95% or more of the projections were formed without being bent.

From the above results, it was found that the precision of shape-transfer from the intaglio plate to the molded product was improved when the molded product, which is formed as the administration section, was removed from the intaglio plate so that removal is carried out in the extending direction of the recess when viewed in the direction perpendicular to the surface of the intaglio plate.

### [Reference Signs List]

10...transdermal administration device, 20...administration section, 21...substrate, 21S...first surface, 21T...second surface, 22,25,26,27...projection, 23A,23D,23F,23H...main lateral face, 23B,23E,23G,23I...auxiliary lateral face, 23C,23J...base, 24a...long side, 24b...short side, 24c,24f,24h...top edge, 24d,24e,24g,24i,24j...lateral edge, 30...adhesive sheet, 31...base sheet, 32...adhesive layer, 50,60,62...intaglio plate, 51,61,63...recess, 120...support casing, 130...adhesive holder, 140...protective film

## Claims

1. A transdermal administration device (10) comprising:
an administration section (20) including
a substrate (21) having a first surface (21S) and a second surface (21T) which is a surface opposite from the first surface, and
a projection (22) which protrudes from the first surface, wherein
the projection has a shape extending along the first surface, and includes:
one linear top edge (24C) which is located away from the first surface, the top edge having a first end and a second end;
two main lateral faces (23A) which have the top edge in common with each other, the two main lateral faces having lateral edges (24d), each lateral edge individually connecting the first end of the top edge and the first surface; and
an auxiliary lateral face (23B) which has the lateral edges in common with the respective main lateral faces and forms one corner together with the two main lateral faces,
an angle made between the two lateral edges on the auxiliary lateral face being an acute angle,
**characterized in that** an angle made between the lateral edge (24d) and the top edge (24c) on the main lateral face (23A) is an obtuse angle.

2. The transdermal administration device according to claim 1, wherein the auxiliary lateral face includes a base side (24b) located within the first surface, and an aspect ratio which is a ratio of a height of the auxiliary lateral face to a length between both ends of the base side is larger than 1.

3. The transdermal administration device according to claim 1 or 2, wherein the auxiliary lateral face is a triangular flat surface having an apex which is the first end of the top edge.

4. The transdermal administration device according to claim 1 or 2, wherein the auxiliary lateral face is a curved surface which curves inward to the projection.

5. The transdermal administration device according to any one of claims 1 to 4, wherein
the auxiliary lateral face is a first auxiliary lateral face,
the lateral edge is a first lateral edge,
the two main lateral faces have lateral edges, each lateral edge individually connecting the second end of the top edge and the first surface, and
the transdermal administration device further includes a second auxiliary lateral face which has the second lateral edges in common with the respective main lateral faces and forms one corner together with the two main lateral faces.

6. The transdermal administration device according to any one of claims 1 to 5, wherein
a direction along which the projection extends is a first direction, the top edge extends along the first direction,
the substrate has a shape extending along the first direction when viewed in a direction perpendicular to the first surface,
the transdermal administration device includes a plurality of the projections, and
the plurality of projections include the plurality of the projections disposed at different positions in the first direction on the first surface.

7. The transdermal administration device according to any one of claims 1 to 5, wherein
a direction along which the projection extends is the first direction,
the top edge extends along the first direction,
the transdermal administration device includes the plurality of the projections,
the plurality of projections include the plurality of the projections disposed at different positions in the first direction on the first surface,
the transdermal administration device further includes an adhesive sheet having an adhesive surface,
the adhesive surface is bonded to the second surface, and
the adhesive surface has a shape extending along the first direction when viewed in the direction perpendicular to the first surface and protrudes outward from the substrate.

8. The transdermal administration device according to claim 7, wherein the substrate has a shape extending along the first direction when viewed in the direction perpendicular to the first surface.

9. A method of producing the transdermal administration device according to claim 1, comprising the steps of: forming a molded product by filling a recess (51) of an intaglio plate (50) with a forming material of the administration section (20), the recess being formed to follow a shape of the projection; and removing the molded product from the intaglio plate so that removal is carried out in an extending direction of the recess when viewed in a direction perpendicular to a surface of the intaglio plate.

## Patentansprüche

1. Transdermale Verabreichungsvorrichtung (10), umfassend:
einen Verabreichungsabschnitt (20) beinhaltend ein Substrat (21) mit einer ersten Oberfläche (21S) und einer zweiten Oberfläche (21T), die eine Oberfläche gegenüber der ersten Oberfläche ist, und
einen Vorsprung (22), der aus der ersten Oberfläche hervorsteht, wobei der Vorsprung eine Form aufweist, die sich entlang der ersten Oberfläche erstreckt, und beinhaltet:
eine lineare Oberkante (24C), die weg von der ersten Oberfläche befindlich ist, wobei die Oberkante ein erstes Ende und ein zweites Ende aufweist;
zwei Hauptseitenflächen (23A), die die Oberkante miteinander gemeinsam haben, wobei die beiden Hauptseitenflächen Seitenkanten (24d) aufweisen, wobei jede Seitenkante individuell das erste Ende der Oberkante und die erste Oberfläche verbindet; und
eine Nebenseitenfläche (23B), die die Seitenkanten mit den jeweiligen Hauptseitenflächen gemeinsam hat und zusammen mit den beiden Hauptseitenflächen eine Ecke bildet,
einen Winkel, der zwischen den beiden Seitenkanten auf der Nebenseitenfläche gebildet ist, der ein spitzer Winkel ist,
**dadurch gekennzeichnet, dass** ein Winkel, der zwischen der Seitenkante (24d) und der Oberkante (24c) auf der Hauptseitenfläche (23A) gebildet ist, ein stumpfer Winkel ist.

2. Transdermale Verabreichungsvorrichtung nach Anspruch 1, wobei die Nebenseitenfläche eine Basisseite (24b) beinhaltet, die sich innerhalb der ersten Oberfläche befindet, und ein Seitenverhältnis, das ein Verhältnis einer Höhe der Nebenseitenfläche zu einer Länge zwischen beiden Enden der Basisseite ist, größer als 1 ist.

3. Transdermale Verabreichungsvorrichtung nach Anspruch 1 oder 2, wobei die Nebenseitenfläche eine dreieckige flache Oberfläche mit einem Scheitel ist, der das erste Ende der Oberkante ist.

4. Transdermale Verabreichungsvorrichtung nach Anspruch 1 oder 2, wobei die Nebenseitenfläche eine gekrümmte Oberfläche ist, die sich nach innen zu dem Vorsprung krümmt.

5. Transdermale Verabreichungsvorrichtung nach einem der Ansprüche 1 bis 4, wobei
die Nebenseitenfläche eine erste Nebenseitenfläche ist,
die Seitenkante eine erste Seitenkante ist,
die beiden Hauptseitenflächen Seitenkanten aufweisen, wobei jede Seitenkante individuell das zweite Ende der Oberkante und die erste Oberfläche verbindet, und
die transdermale Verabreichungsvorrichtung ferner eine zweite Nebenseitenfläche beinhaltet, die die zweiten Seitenkanten mit den jeweiligen Hauptseitenflächen gemeinsam hat und zusammen mit den beiden Hauptseitenflächen eine Ecke bildet.

6. Transdermale Verabreichungsvorrichtung nach einem der Ansprüche 1 bis 5, wobei
eine Richtung, entlang der sich der Vorsprung erstreckt, eine erste Richtung ist, sich die Oberkante entlang der ersten Richtung erstreckt,
das Substrat eine Form aufweist, die sich entlang der ersten Richtung, bei Betrachtung in einer Richtung senkrecht zu der ersten Oberfläche, erstreckt,
die transdermale Verabreichungsvorrichtung eine Mehrzahl der Vorsprünge beinhaltet, und
die Mehrzahl von Vorsprüngen die Mehrzahl der Vorsprünge beinhaltet, die in verschiedenen Positionen in der ersten Richtung auf der ersten Oberfläche angeordnet sind.

7. Transdermale Verabreichungsvorrichtung nach einem der Ansprüche 1 bis 5, wobei
eine Richtung, entlang der sich der Vorsprung erstreckt, die erste Richtung ist, sich die Oberkante entlang der ersten Richtung erstreckt,
die transdermale Verabreichungsvorrichtung die Mehrzahl der Vorsprünge beinhaltet,
die Mehrzahl von Vorsprüngen die Mehrzahl der Vorsprünge beinhaltet, die in verschiedenen Positionen in der ersten Richtung auf der ersten Oberfläche angeordnet sind,
die transdermale Verabreichungsvorrichtung ferner eine Klebefolie mit einer Klebefläche beinhaltet,
die Klebefläche an die zweite Oberfläche gebondet ist, und
die Klebefläche eine Form aufweist, die sich entlang der ersten Richtung, bei Betrachtung in der Richtung senkrecht zu der ersten Oberfläche, erstreckt und nach außen aus dem Substrat hervorsteht.

8. Transdermale Verabreichungsvorrichtung nach Anspruch 7, wobei das Substrat eine Form aufweist, die sich entlang der ersten Richtung, bei Betrachtung in der Richtung senkrecht zu der ersten Oberfläche, erstreckt.

9. Verfahren zum Herstellen der transdermalen Verabreichungsvorrichtung nach Anspruch 1, umfassend die Schritte:
Bilden eines Formteils durch Füllen einer Ausnehmung (51) einer Tiefdruckplatte (50) mit einem Formmaterial des Verabreichungsabschnitts (20), wobei die Ausnehmung dazu ausgebildet ist, einer Form des Vorsprungs zu folgen; und
Entfernen des Formteils aus der Tiefdruckplatte, sodass das Entfernen in einer Erstreckungsrichtung der Ausnehmung, bei Betrachtung in einer Richtung senkrecht zu einer Oberfläche der Tiefdruckplatte, ausgeführt wird.

## Revendications

1. Dispositif d'administration transdermique (10) comprenant :
une section administration (20) comprenant un substrat (21) ayant une première surface (21S) et une seconde surface (21T) qui est une surface opposée à la première surface, et
une saillie (22) qui dépasse de la première surface, dans lequel
la saillie a une forme s'étendant le long de la première surface, et comprend :
un bord supérieur linéaire (24C) qui est éloigné de la première surface, le bord supérieur ayant une première extrémité et une seconde extrémité ;
deux faces latérales principales (23A) qui ont le bord supérieur en commun l'une avec l'autre, les deux faces latérales principales ayant des bords latéraux (24d), chaque bord latéral raccordant individuellement la première extrémité du bord supérieur et la première surface ; et
une face latérale auxiliaire (23B) qui a les bords latéraux en commun avec les faces latérales principales respectives et forme un coin conjointement aux deux faces latérales principales,
un angle formé entre les deux bords latéraux sur la face latérale auxiliaire étant un angle aigu,
**caractérisé en ce qu'**un angle formé entre le bord latéral (24d) et le bord supérieur (24c) sur la face latérale principale (23A) est un angle obtus.

2. Dispositif d'administration transdermique selon la revendication 1, dans lequel la face latérale auxiliaire comprend un côté base (24b) situé à l'intérieur de la première surface, et un rapport d'aspect qui est un rapport d'une hauteur de la face latérale auxiliaire sur une longueur entre les deux extrémités du côté base est supérieur à 1.

3. Dispositif d'administration transdermique selon la revendication 1 ou 2, dans lequel la face latérale auxiliaire est une surface plate triangulaire ayant un apex qui est la première extrémité du bord supérieur.

4. Dispositif d'administration transdermique selon la revendication 1 ou 2, dans lequel la face latérale auxiliaire est une surface incurvée qui s'incurve vers l'intérieur en direction de la saillie.

5. Dispositif d'administration transdermique selon l'une quelconque des revendications 1 à 4, dans lequel
la face latérale auxiliaire est une première face latérale auxiliaire,
le bord latéral est un premier bord latéral,
les deux faces latérales principales ont des bords latéraux, chaque bord latéral raccordant individuellement la seconde extrémité du bord supérieur et la première surface, et
le dispositif d'administration transdermique comprend en outre une seconde face latérale auxiliaire qui a les seconds bords latéraux en commun avec les faces latérales principales respectives et forme un coin conjointement aux deux faces latérales principales.

6. Dispositif d'administration transdermique selon l'une quelconque des revendications 1 à 5, dans lequel
une direction le long de laquelle la saillie s'étend est une première direction,
le bord supérieur s'étend le long de la première direction,
le substrat a une forme s'étendant le long de la première direction lorsqu'il est visualisé dans une direction perpendiculaire à la première surface,
le dispositif d'administration transdermique comprend une pluralité des saillies, et
la pluralité de saillies comprennent la pluralité des saillies disposées au niveau de différentes positions dans la première direction sur la première surface.

7. Dispositif d'administration transdermique selon l'une quelconque des revendications 1 à 5, dans lequel
une direction de long de laquelle la saillie s'étend est la première direction,
le bord supérieur s'étend le long de la première direction,
le dispositif d'administration transdermique comprend la pluralité des saillies,
la pluralité de saillies comprennent la pluralité des saillies disposées au niveau de différentes positions dans la première direction sur la première surface,
le dispositif d'administration transdermique comprend en outre une feuille adhésive ayant une surface adhésive,
la surface adhésive est liée à la seconde surface, et
la surface adhésive a une forme s'étendant le long de la première direction lorsqu'elle est visualisée dans la direction perpendiculaire à la première surface et fait saillie vers l'extérieur depuis le substrat.

8. Dispositif d'administration transdermique selon la revendication 7, dans lequel le substrat a une forme s'étendant le long de la première direction lorsqu'il est visualisé dans la direction perpendiculaire à la première surface.

9. Procédé de production du dispositif d'administration transdermique selon la revendication 1, comprenant les étapes de :
formation d'un produit moulé par remplissage d'une cavité (51) d'une plaque d'impression en creux (50) avec un matériau de formation de la section administration (20), la cavité étant formée pour suivre une forme de la saillie ; et
retrait du produit moulé de la plaque d'impression en creux de sorte que le retrait est réalisé dans une direction d'extension de la cavité lorsqu'elle est visualisée dans une direction perpendiculaire à une surface de la plaque d'impression en creux.
